# EUROPEAN PATENT APPLICATION

(11) **EP 1 541 684 A1**
(43) Date of publication of application: **15.06.2005**
(21) Application number: 03794253.9
(22) Date of filing: 05.09.2003
(51) Int. Cl.: C12N 15/12, C12N 1/21, C12N 5/10

(54) **SMG-1-BINDING PROTEIN AND METHOD OF SCREENING SUBSTANCE CONTROLLING ITS ACTIVITY**

(30) Priority: 05.09.2002 JP 2002260243
(71) Applicant: Japan Science and Technology Agency, Kawaguchi-shi, Saitama 332-0012 (JP); City of Yokohama, Yokohama-shi, Kanagawa 231-0017 (JP)
(72) Inventor: OHNO, Shigeo, Meguro-ku, Tokyo 152-0023 (JP); OHNISHI, Tetsuo, Yokohama-shi, Kanagawa 236-0012 (JP); YAMASHITA, Akio, Yokohama-shi, Kanagawa 235-0033 (JP)
(74) Representative: Minderop, Ralph H., Dr. rer. nat.
(86) International application number: PCT/JP2003/011353
(87) International publication number: WO 2004/022752

(57) **Abstract**

A novel polypeptide binding to SMG-1, a polynucleotide encoding the same, and a system for screening a substance which artificially modifies an mRNA surveillance mechanism, are disclosed. The polypeptide has an activity of binding to SMG-1, an activity of being phosphorylated by SMG-1, and an activity of modifying SMG-1 activities through these activities.

SEQUENCE LISTING

<110>  JAPAN SCIENCE AND TECHNOLOGY CORPORATION
<110>  City of Yokohama

<120>  SMG-1 binding protein and screening method for substances that
can regulate the activity thereof

<130>  JST-699

<150>  JP 2002-260243
<151>  2002-09-05

<160>  8

<170>  PatentIn version 3.1

<210>  1
<211>  3256
<212>  DNA
<213>  Homo sapiens

<220>
<221>  CDS
<222>  (16)..(2991)
<223>

<400>  1
agaacgctct gcact atg gct ggt ccc gtg agc ttg cga gac ctt cta atg      51
                 Met Ala Gly Pro Val Ser Leu Arg Asp Leu Leu Met
                 1               5                   10

gga gcg tca gcc tgg atg ggc tct gaa agt ccc gga ggg tcc cct act      99
Gly Ala Ser Ala Trp Met Gly Ser Glu Ser Pro Gly Gly Ser Pro Thr
        15                  20                  25

gag ggc gga ggg agc gcg gct ggc gga ccg gag cct cca tgg cgg gag      147
Glu Gly Gly Gly Ser Ala Ala Gly Gly Pro Glu Pro Pro Trp Arg Glu
    30                  35                  40

gat gag atc tgc gtt gtg gga atc ttc ggc aag acg gct cta cgc ctg      195
Asp Glu Ile Cys Val Val Gly Ile Phe Gly Lys Thr Ala Leu Arg Leu
45                  50                  55                  60

aat tcc gag aag ttc tct ctt gtg aat acg gtg tgc gac cga cag gtc 243
Asn Ser Glu Lys Phe Ser Leu Val Asn Thr Val Cys Asp Arg Gln Val
                65                  70                  75

ttt cct ctc ttt cgc cac caa gat cct ggg gat cca gga cct gga atc 291
Phe Pro Leu Phe Arg His Gln Asp Pro Gly Asp Pro Gly Pro Gly Ile
            80                  85                  90

aga act gag gct ggc gcc gtg ggt gag gcc ggt gga gcc gag gac cct 339
Arg Thr Glu Ala Gly Ala Val Gly Glu Ala Gly Gly Ala Glu Asp Pro
        95                  100                 105

ggg gct gca gcc ggg ggt tca gtt cgg gga agt gga gct gtc gcg gaa 387
Gly Ala Ala Ala Gly Gly Ser Val Arg Gly Ser Gly Ala Val Ala Glu
    110                 115                 120

ggt aac cga act gag gca ggc tcc cag gac tac agc ctt ctg cag gcc 435
Gly Asn Arg Thr Glu Ala Gly Ser Gln Asp Tyr Ser Leu Leu Gln Ala
125                 130                 135                 140

tac tac agt cag gaa agc aaa gtt ctg tat ctt ctc ctc acc tcc atc 483
Tyr Tyr Ser Gln Glu Ser Lys Val Leu Tyr Leu Leu Leu Thr Ser Ile
                145                 150                 155

tgt gac aat tca cag ctt ctc cgg gct tgt cgg gct ctt cag agc ggg 531
Cys Asp Asn Ser Gln Leu Leu Arg Ala Cys Arg Ala Leu Gln Ser Gly
            160                 165                 170

gaa gct gga ggt gga ctc tct tta cct cat gca gaa gca cac gag ttc 579
Glu Ala Gly Gly Gly Leu Ser Leu Pro His Ala Glu Ala His Glu Phe
        175                 180                 185

tgg aag cat caa gag aag ctg cag tgc ctc agt ctc ctt tac cta ttc 627
Trp Lys His Gln Glu Lys Leu Gln Cys Leu Ser Leu Leu Tyr Leu Phe
    190                 195                 200

tct gtc tgt cat atc ttg ctt ctg gtc cat ccc act tgt tcc ttt gat 675
Ser Val Cys His Ile Leu Leu Leu Val His Pro Thr Cys Ser Phe Asp
205                 210                 215                 220

atc act tat gat cga gta ttc aga gcc ctg gat ggg ctg aga cag aag 723
Ile Thr Tyr Asp Arg Val Phe Arg Ala Leu Asp Gly Leu Arg Gln Lys
                225                 230                 235

gtc ctg ccg ctc ctt aaa aca gcc att aag gat tgt cca gtt ggc aaa 771

Val Leu Pro Leu Leu Lys Thr Ala Ile Lys Asp Cys Pro Val Gly Lys
            240                 245                 250

gac tgg aag cta aac tgc cga cct tgc cca cct aga ctc ctt ttc ctc 819
Asp Trp Lys Leu Asn Cys Arg Pro Cys Pro Pro Arg Leu Leu Phe Leu
        255                 260                 265

ttt caa ctc aat gga gcc ctc aag gta gta cct cct cgg aac caa gac 867
Phe Gln Leu Asn Gly Ala Leu Lys Val Val Pro Pro Arg Asn Gln Asp
    270                 275                 280

cca gct cat cca gac aag ccc aag aaa cat tct ccc aaa agg agg ctg 915
Pro Ala His Pro Asp Lys Pro Lys Lys His Ser Pro Lys Arg Arg Leu
285                 290                 295                 300

cag cat gcc ctg gag gac cag atc tat aga atc ttc cgg aag agt cgt 963
Gln His Ala Leu Glu Asp Gln Ile Tyr Arg Ile Phe Arg Lys Ser Arg
                305                 310                 315

gtc ttg act aat cag agc atc aac tgc ctc ttt act gtg cct gcc aac 1011
Val Leu Thr Asn Gln Ser Ile Asn Cys Leu Phe Thr Val Pro Ala Asn
            320                 325                 330

caa gct ttc gtg tac ata gta ccg gga agc cag gag gag gac cca gta 1059
Gln Ala Phe Val Tyr Ile Val Pro Gly Ser Gln Glu Glu Asp Pro Val
        335                 340                 345

ggt atg ttg ctg gac caa ctt agg agt cat tgt act gtg aag gac ccg 1107
Gly Met Leu Leu Asp Gln Leu Arg Ser His Cys Thr Val Lys Asp Pro
    350                 355                 360

gaa tct ttg ctg gtg cct gca ccc ctt tct ggg cct agg cga tac cag 1155
Glu Ser Leu Leu Val Pro Ala Pro Leu Ser Gly Pro Arg Arg Tyr Gln
365                 370                 375                 380

gtg atg agg cag cac agc cga caa caa ctt tcc ttt cac att gac agc 1203
Val Met Arg Gln His Ser Arg Gln Gln Leu Ser Phe His Ile Asp Ser
                385                 390                 395

agc agt tcc agt tct tca ggc cag cta gtg gat ttc act ctt tgg gaa 1251
Ser Ser Ser Ser Ser Ser Gly Gln Leu Val Asp Phe Thr Leu Trp Glu
            400                 405                 410

ttc cta tgg cag cat gtg gag cta gtt cta agc aag aaa ggt ttc gat 1299
Phe Leu Trp Gln His Val Glu Leu Val Leu Ser Lys Lys Gly Phe Asp

415                    420                    425

gac agt gtg ggc agg aac cca cag cct tcc cat ttt gaa ctt cct act        1347
Asp Ser Val Gly Arg Asn Pro Gln Pro Ser His Phe Glu Leu Pro Thr
    430                    435                    440

tat cag aag tgg atc tca gca gct tca aaa ctg tat gag gtg gct att        1395
Tyr Gln Lys Trp Ile Ser Ala Ala Ser Lys Leu Tyr Glu Val Ala Ile
445                    450                    455                    460

gat ggg aaa gaa gag gac ttg ggg tca ccc act gga gag cta aca tct        1443
Asp Gly Lys Glu Glu Asp Leu Gly Ser Pro Thr Gly Glu Leu Thr Ser
                465                    470                    475

aag att tta agc agt att aaa gtc ttg gaa gga ttt ttg gat att gac        1491
Lys Ile Leu Ser Ser Ile Lys Val Leu Glu Gly Phe Leu Asp Ile Asp
            480                    485                    490

aca aaa ttc tca gaa aac cga tgc caa aaa gct tta ccc atg gcc cac        1539
Thr Lys Phe Ser Glu Asn Arg Cys Gln Lys Ala Leu Pro Met Ala His
        495                    500                    505

agt gcc tac cag tca aat ttg cct cat aat tac aca atg act gtc cat        1587
Ser Ala Tyr Gln Ser Asn Leu Pro His Asn Tyr Thr Met Thr Val His
    510                    515                    520

aag aat cag ctt gcc cag gct ctt cga gtg tac agt caa cat gct aga        1635
Lys Asn Gln Leu Ala Gln Ala Leu Arg Val Tyr Ser Gln His Ala Arg
525                    530                    535                    540

ggt cca gca ttt cac aaa tac gcc atg cag tta cat gag gac tgc tac        1683
Gly Pro Ala Phe His Lys Tyr Ala Met Gln Leu His Glu Asp Cys Tyr
                545                    550                    555

aaa ttt tgg agc aat ggc cat cag ctc tgt gag gag agg agt tta act        1731
Lys Phe Trp Ser Asn Gly His Gln Leu Cys Glu Glu Arg Ser Leu Thr
            560                    565                    570

gat caa cac tgt gtg cac aaa ttt cac tca tta cct aaa tca gga gaa        1779
Asp Gln His Cys Val His Lys Phe His Ser Leu Pro Lys Ser Gly Glu
        575                    580                    585

aaa cca gag gct gat aga aat ccg cct gtg cta tat cac aat agc cga        1827
Lys Pro Glu Ala Asp Arg Asn Pro Pro Val Leu Tyr His Asn Ser Arg
    590                    595                    600

gct cga tct act ggt gct tgc aac tgt gga agg aaa caa gca cct cga     1875
Ala Arg Ser Thr Gly Ala Cys Asn Cys Gly Arg Lys Gln Ala Pro Arg
605                 610                 615                 620

gat gat ccc ttt gat atc aaa gca gcc aat tat gac ttc tat cag ctt     1923
Asp Asp Pro Phe Asp Ile Lys Ala Ala Asn Tyr Asp Phe Tyr Gln Leu
                625                 630                 635

ctg gaa gaa aag tgt tgt gga aaa ttg gat cat atc aat ttc cca gta     1971
Leu Glu Glu Lys Cys Cys Gly Lys Leu Asp His Ile Asn Phe Pro Val
            640                 645                 650

ttt gaa cca agt act cca gat cct gct cct gct aaa aat gaa tcc tct     2019
Phe Glu Pro Ser Thr Pro Asp Pro Ala Pro Ala Lys Asn Glu Ser Ser
        655                 660                 665

cct gct cct cca gat tcg gat gct gat aaa ctt aaa gaa aaa gaa cct     2067
Pro Ala Pro Pro Asp Ser Asp Ala Asp Lys Leu Lys Glu Lys Glu Pro
    670                 675                 680

caa acc caa gga gag agc acg agc ctg agt tta gct ttg agt ttg ggc     2115
Gln Thr Gln Gly Glu Ser Thr Ser Leu Ser Leu Ala Leu Ser Leu Gly
685                 690                 695                 700

caa tcc aca gat agc tta ggt acc tat cca gct gat cca caa gca gga     2163
Gln Ser Thr Asp Ser Leu Gly Thr Tyr Pro Ala Asp Pro Gln Ala Gly
                705                 710                 715

gga gat aat cca gaa gtt cat ggt caa gta gaa gtg aaa act gag aag     2211
Gly Asp Asn Pro Glu Val His Gly Gln Val Glu Val Lys Thr Glu Lys
            720                 725                 730

aga cca aac ttc gtt gat cga cag gca tcc aca gtt gag tat ctc cca     2259
Arg Pro Asn Phe Val Asp Arg Gln Ala Ser Thr Val Glu Tyr Leu Pro
        735                 740                 745

ggc atg cta cat tca aat tgc cct aaa ggt ctc cta ccc aaa ttc tcc     2307
Gly Met Leu His Ser Asn Cys Pro Lys Gly Leu Leu Pro Lys Phe Ser
    750                 755                 760

agc tgg tct ttg gtt aaa cta ggc cct gct aag tct tat aac ttt cat     2355
Ser Trp Ser Leu Val Lys Leu Gly Pro Ala Lys Ser Tyr Asn Phe His
765                 770                 775                 780

aca ggt ttg gac caa cag ggc ttt att cca gga aca aat tat ctt atg 2403
Thr Gly Leu Asp Gln Gln Gly Phe Ile Pro Gly Thr Asn Tyr Leu Met
                785                 790                 795

cct tgg gac att gtc atc agg act aga gct gaa gat gaa gga gac tta 2451
Pro Trp Asp Ile Val Ile Arg Thr Arg Ala Glu Asp Glu Gly Asp Leu
            800                 805                 810

gac aca aac tct tgg cct gct cca aat aaa gct att cct gga aag aga 2499
Asp Thr Asn Ser Trp Pro Ala Pro Asn Lys Ala Ile Pro Gly Lys Arg
        815                 820                 825

agt gcg gtt gta atg gga aga gga aga cgg cga gat gac ata gct cga 2547
Ser Ala Val Val Met Gly Arg Gly Arg Arg Arg Asp Asp Ile Ala Arg
    830                 835                 840

gct ttt gtg ggc ttt gaa tat gaa gac tct cga ggt cgg aga ttc atg 2595
Ala Phe Val Gly Phe Glu Tyr Glu Asp Ser Arg Gly Arg Arg Phe Met
845                 850                 855                 860

tgc tct ggg cct gac aaa gta atg aaa gta atg gga agt ggg cca aag 2643
Cys Ser Gly Pro Asp Lys Val Met Lys Val Met Gly Ser Gly Pro Lys
                865                 870                 875

gaa tca gct tta aaa gcc cta aat agt gac atg ccc tta tat att ctg 2691
Glu Ser Ala Leu Lys Ala Leu Asn Ser Asp Met Pro Leu Tyr Ile Leu
            880                 885                 890

tca tcc tct caa ggt aga ggg ctg aaa cct cat tat gct caa ctt atg 2739
Ser Ser Ser Gln Gly Arg Gly Leu Lys Pro His Tyr Ala Gln Leu Met
        895                 900                 905

agg ctt ttt gtt gtg gtt cct gat gct cct ttg cag ata ata cta atg 2787
Arg Leu Phe Val Val Val Pro Asp Ala Pro Leu Gln Ile Ile Leu Met
    910                 915                 920

cct cag gtt cag cca ggc cca cca cca tgt ccg gta ttc tac cca gaa 2835
Pro Gln Val Gln Pro Gly Pro Pro Pro Cys Pro Val Phe Tyr Pro Glu
925                 930                 935                 940

aaa caa gaa atc acc ctt cca cct gat ggc ctt tgg gtt ttg aga ttt 2883
Lys Gln Glu Ile Thr Leu Pro Pro Asp Gly Leu Trp Val Leu Arg Phe
                945                 950                 955

cct tat gca tat gtg act gag aga gga cct tgt ttc ccc cct aag gaa 2931

Pro Tyr Ala Tyr Val Thr Glu Arg Gly Pro Cys Phe Pro Pro Lys Glu
            960                 965                 970

aat gtg cag tta atg agc tac aag gtg ctc cgt ggg gtt ctt aag gca 2979
Asn Val Gln Leu Met Ser Tyr Lys Val Leu Arg Gly Val Leu Lys Ala
        975                 980                 985

gta aca caa taa gtgttttcca gccagttcaa tcctatactt gagctggttt 3031
Val Thr Gln
    990

ttgtttttgg tatttgtggc tgtgtttttg gtatgtgttt actgtgtttt cccaaatcca 3091

aaatgtgttt tggttacagg agttcagtat ttggaaacta atttgttcta ctttttcatt 3151

atattgttga tatttattgt aaacgtgact acaactttct aataaagatt ggttgttcta 3211

gaaaagaata taaaatacat ggatgataaa aaaaaaaaaa aaaaa 3256

<210> 2
<211> 991
<212> PRT
<213> Homo sapiens

<400> 2

Met Ala Gly Pro Val Ser Leu Arg Asp Leu Leu Met Gly Ala Ser Ala
1               5                   10                  15

Trp Met Gly Ser Glu Ser Pro Gly Gly Ser Pro Thr Glu Gly Gly Gly
            20                  25                  30

Ser Ala Ala Gly Gly Pro Glu Pro Pro Trp Arg Glu Asp Glu Ile Cys
        35                  40                  45

Val Val Gly Ile Phe Gly Lys Thr Ala Leu Arg Leu Asn Ser Glu Lys
    50                  55                  60

Phe Ser Leu Val Asn Thr Val Cys Asp Arg Gln Val Phe Pro Leu Phe
65                  70                  75                  80

Arg His Gln Asp Pro Gly Asp Pro Gly Pro Gly Ile Arg Thr Glu Ala
                85                  90                  95

Gly Ala Val Gly Glu Ala Gly Gly Ala Glu Asp Pro Gly Ala Ala Ala
            100                 105                 110

Gly Gly Ser Val Arg Gly Ser Gly Ala Val Ala Glu Gly Asn Arg Thr
        115                 120                 125

Glu Ala Gly Ser Gln Asp Tyr Ser Leu Leu Gln Ala Tyr Tyr Ser Gln
    130                 135                 140

Glu Ser Lys Val Leu Tyr Leu Leu Leu Thr Ser Ile Cys Asp Asn Ser
145                 150                 155                 160

Gln Leu Leu Arg Ala Cys Arg Ala Leu Gln Ser Gly Glu Ala Gly Gly
                165                 170                 175

Gly Leu Ser Leu Pro His Ala Glu Ala His Glu Phe Trp Lys His Gln
            180                 185                 190

Glu Lys Leu Gln Cys Leu Ser Leu Leu Tyr Leu Phe Ser Val Cys His
        195                 200                 205

Ile Leu Leu Leu Val His Pro Thr Cys Ser Phe Asp Ile Thr Tyr Asp
    210                 215                 220

Arg Val Phe Arg Ala Leu Asp Gly Leu Arg Gln Lys Val Leu Pro Leu
225                 230                 235                 240

Leu Lys Thr Ala Ile Lys Asp Cys Pro Val Gly Lys Asp Trp Lys Leu
                245                 250                 255

Asn Cys Arg Pro Cys Pro Pro Arg Leu Leu Phe Leu Phe Gln Leu Asn
            260                 265                 270

Gly Ala Leu Lys Val Val Pro Pro Arg Asn Gln Asp Pro Ala His Pro
        275                 280                 285

Asp Lys Pro Lys Lys His Ser Pro Lys Arg Arg Leu Gln His Ala Leu
    290                 295                 300

Glu Asp Gln Ile Tyr Arg Ile Phe Arg Lys Ser Arg Val Leu Thr Asn
305                 310                 315                 320

Gln Ser Ile Asn Cys Leu Phe Thr Val Pro Ala Asn Gln Ala Phe Val
                325                 330                 335

Tyr Ile Val Pro Gly Ser Gln Glu Glu Asp Pro Val Gly Met Leu Leu
            340                 345                 350

Asp Gln Leu Arg Ser His Cys Thr Val Lys Asp Pro Glu Ser Leu Leu
        355                 360                 365

Val Pro Ala Pro Leu Ser Gly Pro Arg Arg Tyr Gln Val Met Arg Gln
    370                 375                 380

His Ser Arg Gln Gln Leu Ser Phe His Ile Asp Ser Ser Ser Ser Ser
385                 390                 395                 400

Ser Ser Gly Gln Leu Val Asp Phe Thr Leu Trp Glu Phe Leu Trp Gln
                405                 410                 415

His Val Glu Leu Val Leu Ser Lys Lys Gly Phe Asp Asp Ser Val Gly
            420                 425                 430

Arg Asn Pro Gln Pro Ser His Phe Glu Leu Pro Thr Tyr Gln Lys Trp
        435                 440                 445

Ile Ser Ala Ala Ser Lys Leu Tyr Glu Val Ala Ile Asp Gly Lys Glu
    450                 455                 460

Glu Asp Leu Gly Ser Pro Thr Gly Glu Leu Thr Ser Lys Ile Leu Ser
465                 470                 475                 480

Ser Ile Lys Val Leu Glu Gly Phe Leu Asp Ile Asp Thr Lys Phe Ser
                485                 490                 495

Glu Asn Arg Cys Gln Lys Ala Leu Pro Met Ala His Ser Ala Tyr Gln
            500                 505                 510

Ser Asn Leu Pro His Asn Tyr Thr Met Thr Val His Lys Asn Gln Leu
        515                 520                 525

Ala Gln Ala Leu Arg Val Tyr Ser Gln His Ala Arg Gly Pro Ala Phe
    530                 535                 540

His Lys Tyr Ala Met Gln Leu His Glu Asp Cys Tyr Lys Phe Trp Ser
545                 550                 555                 560

Asn Gly His Gln Leu Cys Glu Glu Arg Ser Leu Thr Asp Gln His Cys
                565                 570                 575

Val His Lys Phe His Ser Leu Pro Lys Ser Gly Glu Lys Pro Glu Ala
            580                 585                 590

Asp Arg Asn Pro Pro Val Leu Tyr His Asn Ser Arg Ala Arg Ser Thr
        595                 600                 605

Gly Ala Cys Asn Cys Gly Arg Lys Gln Ala Pro Arg Asp Asp Pro Phe

    610                 615                 620

Asp Ile Lys Ala Ala Asn Tyr Asp Phe Tyr Gln Leu Leu Glu Glu Lys
625                 630                 635                 640

Cys Cys Gly Lys Leu Asp His Ile Asn Phe Pro Val Phe Glu Pro Ser
                645                 650                 655

Thr Pro Asp Pro Ala Pro Ala Lys Asn Glu Ser Ser Pro Ala Pro Pro
            660                 665                 670

Asp Ser Asp Ala Asp Lys Leu Lys Glu Lys Glu Pro Gln Thr Gln Gly
        675                 680                 685

Glu Ser Thr Ser Leu Ser Leu Ala Leu Ser Leu Gly Gln Ser Thr Asp
    690                 695                 700

Ser Leu Gly Thr Tyr Pro Ala Asp Pro Gln Ala Gly Gly Asp Asn Pro
705                 710                 715                 720

Glu Val His Gly Gln Val Glu Val Lys Thr Glu Lys Arg Pro Asn Phe
                725                 730                 735

Val Asp Arg Gln Ala Ser Thr Val Glu Tyr Leu Pro Gly Met Leu His
            740                 745                 750

Ser Asn Cys Pro Lys Gly Leu Leu Pro Lys Phe Ser Ser Trp Ser Leu
        755                 760                 765

Val Lys Leu Gly Pro Ala Lys Ser Tyr Asn Phe His Thr Gly Leu Asp
    770                 775                 780

Gln Gln Gly Phe Ile Pro Gly Thr Asn Tyr Leu Met Pro Trp Asp Ile
785                 790                 795                 800

Val Ile Arg Thr Arg Ala Glu Asp Glu Gly Asp Leu Asp Thr Asn Ser
                805                 810                 815

Trp Pro Ala Pro Asn Lys Ala Ile Pro Gly Lys Arg Ser Ala Val Val
            820                 825                 830

Met Gly Arg Gly Arg Arg Arg Asp Asp Ile Ala Arg Ala Phe Val Gly
        835                 840                 845

Phe Glu Tyr Glu Asp Ser Arg Gly Arg Arg Phe Met Cys Ser Gly Pro
    850                 855                 860

Asp Lys Val Met Lys Val Met Gly Ser Gly Pro Lys Glu Ser Ala Leu
865                 870                 875                 880

Lys Ala Leu Asn Ser Asp Met Pro Leu Tyr Ile Leu Ser Ser Ser Gln
                885                 890                 895

Gly Arg Gly Leu Lys Pro His Tyr Ala Gln Leu Met Arg Leu Phe Val
            900                 905                 910

Val Val Pro Asp Ala Pro Leu Gln Ile Ile Leu Met Pro Gln Val Gln
        915                 920                 925

Pro Gly Pro Pro Pro Cys Pro Val Phe Tyr Pro Glu Lys Gln Glu Ile
    930                 935                 940

Thr Leu Pro Pro Asp Gly Leu Trp Val Leu Arg Phe Pro Tyr Ala Tyr
945                 950                 955                 960

Val Thr Glu Arg Gly Pro Cys Phe Pro Pro Lys Glu Asn Val Gln Leu
                965                 970                 975

Met Ser Tyr Lys Val Leu Arg Gly Val Leu Lys Ala Val Thr Gln
            980                 985                 990

<210> 3
<211> 7
<212> PRT
<213> Homo sapiens

<400> 3

Val Leu Arg Gly Val Leu Lys
1               5

<210> 4
<211> 9
<212> PRT
<213> Homo sapiens

<400> 4

Thr Ala Leu Arg Leu Asn Ser Glu Lys
1               5

<210> 5
<211> 8
<212> PRT
<213> Homo sapiens

<400> 5

Phe Ser Glu Asn Arg Cys Gln Lys
1               5

<210> 6
<211> 11
<212> PRT
<213> Homo sapiens

<400> 6

Gln Ala Pro Arg Asp Asp Pro Phe Asp Ile Lys
1               5                   10

<210>  7
<211>  21
<212>  PRT
<213>  Homo sapiens

<400>  7

Asn Gln Leu Ala Gln Ala Leu Arg Val Tyr Ser Gln His Ala Arg Gly
1               5                   10                  15

Pro Ala Phe His Lys
            20

<210>  8
<211>  22
<212>  PRT
<213>  Homo sapiens

<400>  8

Gly Phe Asp Asp Ser Val Gly Arg Asn Pro Gln Pro Ser His Phe Glu
1               5                   10                  15

Leu Pro Thr Tyr Gln Lys
            20

## Description

### TECHNICAL FIELD

The present invention relates to an SMG-1 binding protein and a screening method for substances that can regulate or modify the activity thereof.

### BACKGROUND ART

Approximately one-quarter of abnormal phenotypes due to gene mutations are thought to be caused by a nonsense codon generated in a coding region, due to one or more mutations such as substitution, recombination, and/or deletion in the nucleotide sequence of the corresponding gene. The gene with such a nonsense codon encodes an abnormal protein lacking the C-terminal region, but no mRNA encoding the abnormal protein is usually detected. This is because cells have a mechanism in which mRNAs having one or more nonsense codons at abnormal positions are recognized and removed. The mechanism is called nonsense-mediated mRNA decay (or mRNA surveillance), and it is suggested that the mechanism is closely involved in human genetic diseases, and plays a physiological role in the removal of abnormal mRNAs generated when immunocompetent cells are matured. However, the molecular mechanism has not been clarified in vertebrates.

The present inventors and coworkers revealed and reported that phosphorylation of human UPF1 by a protein kinase molecule, human SMG-1 (hSMG-1), is essential to a process of mRNA surveillance [Yamashita, Akio, et al, "GENES & DEVELOPMENT", (USA), 2001, 15, p. 2215-2228 (non patent reference 1)]. However, a surveillance complex containing human SMG-1 has not been clarified.

In this connection, a nucleotide sequence of a human cDNA encoding a novel polypeptide found in the present invention was reported in Kawabata, A., Hikiji, T., Kobatake, N., Inagaki, H., Ikema, Y., Okamoto, S., Okitani, R., Ota, T., Suzuki, Y., Obayashi, M., Nishi, T., Shibahara, T., Tanaka, T., Nakamura, Y., Isogai, T., and Sugano, S., "NEDO human cDNA sequencing project." Homo sapiens cDNA...[gi:10439815]. Datasheet. [online]. Entrez Nucleotides database, National Center for Biotechnology Information. Entrez accession No: AK026858. [retrieved on 2002-09-02]. Retrieved from the Internet: <URL: http://www.ncbi.nlm.nih.gov:80/entrez/query.fcgi?cmd=Retrieve&db=nucleotide&list_uids=10439815&dop t=GenBank> (non patent reference 2). However, the reference does not disclose that the polypeptide was obtained by expressing the cDNA, and does not disclose the functions of the polypeptide.

Further, protein p140 having a molecular weight similar to that of the novel polypeptide found in the present invention was disclosed in Onishi, et al., "Analysis of structure and functions of novel PI3K-related huge protein kinase", "PROGRAM AND ABSTRACTS IN THE 22ND ANNUAL MEETING OF THE MOLECULAR BIOLOGY SOCIETY OF JAPAN", 1999, p. 235 (non patent reference 3). However, the reference merely discloses human phosphatidyl inositol kinase (PIK)-related protein kinase (PIKK), which plays an important role in cell cycle progression or a regulation of checkpoints, but neither discloses nor suggests the mRNA surveillance mechanism.
(non patent reference 1) Yamashita, Akio, et al, "GENES & DEVELOPMENT", (USA), 2001, 15, p. 2215-2228
(non patent reference 2) Kawabata, A., Hikiji, T., Kobatake, N., Inagaki, H., Ikema, Y., Okamoto, S., Okitani, R., Ota, T., Suzuki, Y., Obayashi, M., Nishi, T., Shibahara, T., Tanaka, T., Nakamura, Y., Isogai, T., and Sugano, S., "NEDO human cDNA sequencing project." Homo sapiens cDNA...[gi: 10439815]. Datasheet. [online]. Entrez Nucleotides database, National Center for Biotechnology Information. Entrez accession No: AK026858. [retrieved on 2002-09-02].
   Retrieved from the Internet: <URL: http://www.ncbi.nlm.nih. gov:80/entrez/query.fcgi?cmd=Retrieve&db=nucleotide&list_uid s=10439815&dopt=GenBank>
(non patent reference 3) Onishi, et al., "Analysis of structure and functions of novel PI3K-related huge protein kinase", "PROGRAM AND ABSTRACTS IN THE 22ND ANNUAL MEETING OF THE MOLECULAR BIOLOGY SOCIETY OF JAPAN", 1999, p. 235

### DISCLOSURE OF INVENTION

The present inventors engaged in intensive search with the object of elucidating an mRNA surveillance mechanism and developing a method capable of artificially modifying the mRNA surveillance mechanism, and as a result, identified a novel protein firmly binding to hSMG-1, and determined the full-length sequence thereof. The molecule firmly binds to hSMG-1 in cells, and thus, it is predicted that the molecule is closely involved in the modification of a kinase activity, intracellular localization, and the modification of an activity of a surveillance complex. Therefore, the present inventors found that the novel protein is a candidate for the molecule target to artificially modify the mRNA surveillance mechanism. The present invention is based on these findings.

Therefore, an object of the present invention is to provide a novel polypeptide which binds to SMG-1, a polynucleotide encoding the polypeptide, and a system for screening substances which artificially modify the mRNA surveillance mechanism.

The present invention relates to (1) a polypeptide consisting of the amino acid sequence of SEQ ID NO: 2, (2) a polypeptide exhibiting an SMGBP1 activity and comprising the amino acid sequence of SEQ ID NO: 2, (3) a polypeptide exhibiting an SMGBP1 activity and comprising an amino acid sequence in which one or plural amino acids are deleted, substituted, and/or inserted at one or plural positions in the amino acid sequence of SEQ ID NO: 2, or (4) a polypeptide exhibiting an SMGBP1 activity and comprising an amino acid sequence having a 90% or more homology with that of SEQ ID NO: 2.

Further, the present invention relates to a polynucleotide encoding the polypeptide.

Further, the present invention relates to an expression vector comprising the polynucleotide.

Further, the present invention relates to a transformant comprising the polynucleotide.

Further, the present invention relates to an antibody or a fragment thereof, which binds to the above polypeptide.

Further, the present invention relates to a knockout non-human animal or a cell thereof, wherein an expression of a gene encoding the above polypeptide is partially or completely suppressed.

Further, the present invention relates to a method for screening a substance which modifies an SGMBP1 activity of the above polypeptide, comprising the steps of:
(1) bringing the polypeptide and SMG-1 into contact with a substance to be tested, under conditions such that the polypeptide exhibits the SMGBP1 activity in the absence of the substance to be tested, and
(2) analyzing whether or not the polypeptide exhibits the SMGBP1 activity.

Further, the present invention relates to a method for screening a substance which modifies an SGMBP1 activity of the above polypeptide, comprising the steps of:
(1) bringing a cell capable of producing the above polypeptide and SMG-1 into contact with a substance to be tested, under conditions such that the cell produces the polypeptide in the absence of the substance to be tested, and
(2) analyzing whether or not the polypeptide exhibits the SMGBP1 activity.

Further, the present invention relates to an agent for suppressing nonsense-mediated mRNA decay, comprising, as an active ingredient, a substance which modifies an SMGBP1 activity of the above polypeptide (for example, a substance obtained by the above screening method and modifying an SMGBP1 activity of the above polypeptide).

Further, the present invention relates to an agent for treating and/or preventing a disease caused by a premature translation termination codon generated by a nonsense mutation, comprising, as an active ingredient, a substance which modifies an SMGBP1 activity of the above polypeptide (for example, a substance obtained by the above screening method and modifying an SMGBP1 activity of the above polypeptide).

Further, the present invention relates to an agent for promoting nonsense-mediated mRNA decay, comprising, as an active ingredient, a substance which modifies an SMGBP1 activity of the above polypeptide (for example, a substance obtained by the above screening method and modifying an SMGBP1 activity of the above polypeptide).

Further, the present invention relates to a method for suppressing nonsense-mediated mRNA decay, comprising administering to a subject in need thereof a substance which modifies an SMGBP1 activity of the above polypeptide, in an amount effective therefor.

Further, the present invention relates to a method for treating and/or preventing a disease caused by a premature translation termination codon generated by a nonsense mutation, comprising administering to a subject in need thereof a substance which modifies an SMGBP1 activity of the above polypeptide, in an amount effective therefor.

Further, the present invention relates to a method for promoting nonsense-mediated mRNA decay, comprising administering to a subject in need thereof a substance which modifies an SMGBP1 activity of the above polypeptide, in an amount effective therefor.

Further, the present invention relates to use of a substance which modifies an SMGBP1 activity of the above polypeptide, in the manufacture of an agent for suppressing nonsense-mediated mRNA decay.

Further, the present invention relates to use of a substance which modifies an SMGBP1 activity of the above polypeptide, in the manufacture of an agent for treating and/or preventing a disease caused by a premature translation termination codon generated by a nonsense mutation.

Further, the present invention relates to use of a substance which modifies an SMGBP1 activity of the above polypeptide, in the manufacture of an agent for promoting nonsense-mediated mRNA decay.

### BRIEF DESCRIPTION OF THE DRAWINGS

Figure 1 is an autoradiogram showing the results in which immunoprecipitates of the human HeLa cell extract obtained by using the anti-hSMG-1 antiserum (antiserum C) were phosphorylated.
Figure 2 is an autoradiogram showing the results in which immunoprecipitates of the human HeLa cell extract obtained by using two anti-hSMG-1 antisera (antiserum C or antiserum L) were phosphorylated.
Figure 3 is a chromatogram of the human HeLa cell extract with anion exchange column HiTrapQ.
Figure 4 is a drawing showing the results of Western blotting performed to show the distribution of human SMG-1 in the fractions described in Figure 3.
Figure 5 is an autoradiograph showing the results in which immunoprecipitates of the fractions described in Figure 3 obtained by using the hSMG-1 serum were phosphorylated.
Figure 6 is a drawing showing the results in which immunoprecipitates of fractions 20 to 31 described in Figure 3 obtained by using the hSMG-1 serum were phosphorylated, and subjected to Western blotting.
Figure 7 is a drawing showing the results in which immunoprecipitates of the human HeLa cell extract obtained by using the anti-hSMG-1 IgG (antibody N) were subjected to SDS-PAGE, and stained by silver staining.
Figure 8 illustrates the primary structure of the translated product of AK026858.1, and the positions of sequences corresponding to AP1, AP2, AP3, AP4, AP5, and AP8.
Figure 9 is a drawing showing the results in which immunoprecipitates of the human HeLa cell extract obtained by using the anti-hSMG-1 antisera (antiserum N, antiserum L, or antiserum C) were subjected to Western blotting using the anti AK026858.1-translated-product antibody.

### BEST MODE FOR CARRYING OUT THE INVENTION

The present invention will be explained in detail hereinafter.

### [1] Polypeptide of the present invention

The polypeptide of the present invention includes
(1) a polypeptide consisting of the amino acid sequence of SEQ ID NO: 2;
(2) a polypeptide exhibiting an SMGBP1 activity and comprising the amino acid sequence of SEQ ID NO: 2;
(3) a polypeptide exhibiting an SMGBP1 activity and comprising an amino acid sequence in which one or plural amino acids are deleted, substituted, and/or inserted at one or plural positions in the amino acid sequence of SEQ ID NO: 2 (hereinafter referred to as a functionally equivalent mutant); and
(4) a polypeptide exhibiting an SMGBP1 activity and comprising an amino acid sequence having a 90% or more homology with that of SEQ ID NO: 2, (hereinafter referred to as a homologous polypeptide).

The "polypeptide consisting of the amino acid sequence of SEQ ID NO: 2" as the polypeptide of the present invention is a novel protein, which consists of 991 amino acid residues, and may bind to SMG-1 (particularly human SMG-1) to form a complex. The present inventors named the polypeptide "SMGBP1" (SMG Binding Protein 1) on the basis of the activity of binding to SMG-1.

The term "SMGBP1 activity" as used herein means an activity of binding SMG-1 (particularly human SMG-1), an activity of being phosphorylated by SMG-1, and/or an activity of modifying SMG-1 activities through these activities. Further, "exhibiting an SMGBP1 activity" as used herein means that a polypeptide exhibits at least one of these activities.

In this connection, SMG-1 is phosphatidyl inositol kinase (PIK) related protein kinase (PIKK) that is essential to a process of mRNA surveillance, and has an autophosphorylation activity. Further, SMG-1 has an activity of phosphorylating UPF1/SMG-2 [Sun, X. et al., Proc. Natl. Acad. Sci. USA, 1998, 95, p. 10009-100014; and Bhattacharya, A. et al., Rna, 2000, 6, p. 1226-1235] essential to nonsense-mediated mRNA decay (NMD), and modifies NMD positively [Yamashita, Akio. et al., "GENES & DEVELOPMENT", (USA), 2001, 15, p. 2215-2228].

A method for confirming whether or not a polypeptide to be tested "exhibits an SMGBP1 activity" is not particularly limited. It may be confirmed, for example, by bringing the test polypeptide into contact with SMG-1 (for example, human SMG-1), and analyzing whether or not a complex of the test polypeptide and SMG-1 is formed.

As a method for bringing the test polypeptide into contact with SMG-1 in the above confirmation method, there may be mentioned, for example,
a method in which the test polypeptide and SMG-1 are separately prepared by genetic engineering techniques, and are brought into contact with each other; or
a method in which a polynucleotide encoding the test polypeptide is introduced into cells capable of producing SMG-1, and the test polypeptide and SMG-1 are produced and brought into contact with each other in the cells.

As a method for analyzing the complex of the test polypeptide and SMG-1 in the above confirmation method, there may be mentioned, for example,
a method in which immunoprecipitation is performed using an antibody against either of two polypeptides, i.e., SMG-1 and the test polypeptide, (for example, anti-SMG-1 antibody), and the resulting immunoprecipitate is analyzed by Western blotting using an antibody against the other polypeptide (for example, anti-test-polypeptide antibody) to confirm the existence of the latter polypeptide [for example, a method described in Example 4 (2)];
a method in which immunoprecipitation is performed using an antibody against either SMG-1 or the test polypeptide (for example, anti-SMG-1 antibody), and the resulting immunoprecipitate is electrophoresed and analyzed by an appropriate detection method (for example, silver staining) to confirm the existence of the other polypeptide (for example, the test polypeptide) [for example, a method described in Example 3 (1)];
a method in which immunoprecipitation is performed using an anti-SMG-1 antibody, and the resulting immunoprecipitate is phosphorylated under the conditions such that phosphorylation by SMG-1 may be carried out to confirm the existence of the phosphorylated test polypeptide [for example, a method described in Example 1(2) to Example 1(4)]; or
a method in which chromatography is carried out to confirm whether or not the test polypeptide and SMG-1 exist in the same fraction [for example, a method described in Example 2(2) to Example 2(3)].

The "polypeptide comprising the amino acid sequence of SEQ ID NO: 2, and exhibiting an SMGBP1 activity" as the polypeptide of the present invention may be, for example, a fusion polypeptide consisting of an amino acid sequence in which an appropriate marker sequence or the like is added to the N-terminus and/or the C-terminus of the amino acid sequence of SEQ ID NO: 2, and exhibiting an SMG-1 activity.

As the marker sequence in the polypeptide of the present invention, for example, a sequence for easily carrying out confirmation of polypeptide expression, confirmation of intracellular localization thereof, purification thereof, or the like may be used. As the sequence, there may be mentioned, for example, the FLAG tag, the hexa-histidine tag, the hemagglutinin tag, the myc epitope, or the like.

The functionally equivalent mutant of the present invention is not particularly limited, so long as it is a polypeptide comprising an amino acid sequence in which one or plural (preferably 1 to 10, more preferably 1 to 7, most preferably 1 to 5) amino acids, such as one to several amino acids, are deleted, substituted, and/or inserted at one or plural positions of the amino acid sequence of SEQ ID NO: 2, and exhibiting an SMGBP1 activity. Further, an origin of the functionally equivalent mutant is not limited to a human.

The functionally equivalent mutant of the present invention includes, for example, human mutants of the polypeptide (i.e., human SMGBP1) consisting of the amino acid sequence of SEQ ID NO: 2, and functionally equivalent mutants derived from organisms other than human (such as simian, mouse, rat, hamster, or dog). Further, the functionally equivalent mutant of the present invention includes polypeptides prepared using polynucleotides obtained by artificially modifying polynucleotides encoding these native polypeptides (i.e., human mutants or functionally equivalent mutants derived from organisms other than human) or polynucleotides encoding the polypeptide consisting of the amino acid sequence of SEQ ID NO: 2 by genetic engineering techniques.

Human mutants of the polypeptide consisting of the amino acid sequence of SEQ ID NO: 2 or functionally equivalent mutants derived from organisms other than a human may be obtained by those skilled in the art in accordance with the information of a nucleotide sequence (for example, the nucleotide sequence consisting of 16th to 2988th bases in the nucleotide sequence of SEQ ID NO: 1) of a polynucleotide encoding the polypeptide consisting of the amino acid sequence of SEQ ID NO: 2. In this connection, genetic engineering techniques may be generally performed in accordance with known methods (for example, Sambrook, J. et al., "Molecular Cloning-A Laboratory Manual", Cold Spring Harbor Laboratory, NY, 1989).

For example, an appropriate probe or appropriate primers are designed in accordance with the information of a nucleotide sequence of a polynucleotide encoding the polypeptide consisting of the amino acid sequence of SEQ ID NO: 2. A polymerase chain reaction (PCR) method (Saiki, R. K. et al., Science, 239, 487-491, 1988) or a hybridization method is carried out using a sample (for example, total RNA or an mRNA fraction, a cDNA library, or a phage library) prepared from an organism (for example, a mammal such as human, simian, mouse, rat, hamster, or dog) of interest and the primers or the probe to obtain a polynucleotide encoding the polypeptide. A desired polypeptide may be obtained by expressing the resulting polynucleotide in an appropriate expression system and confirming that the expressed polypeptide exhibits an SMGBP1 activity.

Further, the polypeptide artificially modified by genetic engineering techniques may be obtained by, for example, the following procedure. A polynucleotide encoding the polypeptide may be obtained by a conventional method, for example, site-directed mutagenesis (Mark, D. F. et al., Proc. Natl. Acad. Sci. USA, 81, 5662-5666, 1984). A desired polypeptide may be obtained by expressing the resulting polynucleotide in an appropriate expression system and confirming that the expressed polypeptide exhibits an SMGBP1 activity.

The homologous polypeptide of the present invention is not particularly limited, so long as it comprises an amino acid sequence having a 90% or more homology with the amino acid sequence of SEQ ID NO: 2, and exhibits an SMG-1 activity. The homologous polypeptide of the present invention may comprise an amino acid sequence having preferably a 95% or more homology, more preferably a 98% or more homology, most preferably a 99% or more homology, with respect to the amino acid sequence of SEQ ID NO: 2.

The term "homology" as used herein means a value obtained by BLAST [Basic local alignment search tool; Altschul, S. F. et al., J. Mol. Biol., 215, 403-410, (1990)].

### [2] Polynucleotide of the present invention

The polynucleotide of the present invention is not particularly limited, so long as it encodes the polypeptide of the present invention. As the polynucleotide of the present invention, there may be mentioned, for example, a polynucleotide comprising the nucleotide sequence consisting of the 16th to 2988th bases in the nucleotide sequence of SEQ ID NO: 1, or a polynucleotide consisting of the nucleotide sequence consisting of the 16th to 2988th bases in the nucleotide sequence of SEQ ID NO: 1. The term "polynucleotide" as used herein includes both DNA and RNA.

A method for producing the polynucleotide of the present invention is not particularly limited, but there may be mentioned, for example, (1) a method using PCR, (2) a method using conventional genetic engineering techniques (i.e., a method for selecting a transformant comprising a desired cDNA from strains transformed with a cDNA library), or (3) a chemical synthesis method. These methods will be explained in this order hereinafter.

In the method using PCR of the item (1), the polynucleotide of the present invention may be produced, for example, by the following procedure.

mRNA is extracted from human cells or tissue capable of producing the polypeptide of the present invention. A pair of primers, between which full-length mRNA corresponding to the polypeptide of the present invention or a partial region of the mRNA is located, is synthesized on the basis of the nucleotide sequence of a polynucleotide encoding the polypeptide of the present invention. Full-length cDNA encoding the polypeptide of the present invention or a part of the cDNA may be obtained by performing a reverse transcriptase-polymerase chain reaction (RT-PCR) using the extracted mRNA as a template.

More particularly, total RNA containing mRNA encoding the polypeptide of the present invention is extracted by a known method from cells or tissue capable of producing the polypeptide of the present invention. As an extraction method, there may be mentioned, for example, a guanidine thiocyanate-hot phenol method, a guanidine thiocyanate-guanidine hydrochloride method, or a guanidine thiocyanate-cesium chloride method. The guanidine thiocyanate-cesium chloride method is preferably used. The cells or tissue capable of producing the polypeptide of the present invention may be identified, for example, by a Northern blotting method using a polynucleotide or a part thereof encoding the polypeptide of the present invention or a Western blotting method using an antibody specific for the polypeptide of the present invention.

Next, the extracted mRNA is purified. Purification of the mRNA may be made in accordance with a conventional method. For example, the mRNA may be purified by adsorption and elution using an oligo (dT)-cellulose column. The mRNA may be further fractionated by, for example, a sucrose density gradient centrifugation, if necessary. Alternatively, commercially available extracted and purified mRNA may be used without carrying out the extraction of the mRNA.

Next, the first-strand cDNA is synthesized by carrying out a reverse transcriptase reaction of the purified mRNA in the presence of a random primer, an oligo dT primer, and/or a custom primer. This synthesis may be carried out in accordance with a conventional method. The resulting first-strand cDNA is subjected to PCR using two primers between which a full-length or a partial region of the polynucleotide of interest is located, thereby amplifying the cDNA of interest. The resulting DNA is fractionated by, for example, an agarose gel electrophoresis. The DNA fragment of interest may be obtained by carrying out a digestion of the DNA with restriction enzymes and subsequent ligation, if necessary.

In the method using conventional genetic engineering techniques of the item (2), the polynucleotide of the present invention may be produced, for example, by the following procedure.

First, single-stranded cDNA is synthesized by using reverse transcriptase from mRNA prepared by the above-mentioned PCR method as a template, and then double-stranded cDNA is synthesized from the single-stranded cDNA. As this method, there may be mentioned, for example, an S1 nuclease method (Efstratiadis, A. et al., Cell, 7, 279-288, 1976), a Land method (Land, H. et al., Nucleic Acids Res., 9, 2251-2266, 1981), an O. Joon Yoo method (Yoo, O. J. et al., Proc. Natl. Acad. Sci. USA, 79, 1049-1053, 1983), and an Okayama-Berg method (Okayama, H. and Berg, P., Mol. Cell. Biol., 2, 161-170, 1982).

Next, a recombinant plasmid comprising the double-stranded cDNA is prepared and introduced into an Escherichia coli strain, such as DH 5α, HB101, or JM109, thereby transforming the strain. A transformant is selected using a drug resistance against, for example, tetracycline, ampicillin, or kanamycin as a marker. When the host cell is E. coli, transformation of the host cell may be carried out, for example, by the method of Hanahan (Hanahan, D. J., Mol. Biol., 166, 557-580, 1983); namely, a method in which the recombinant DNA is added to competent cells prepared in the presence of CaCl₂, MgCl₂, or RbCl. Further, as a vector other than a plasmid, a phage vector such as a lambda system may be used.

As a method for selecting a transformant containing the cDNA of interest from the resulting transformants, various methods such as (i) a method for screening a transformant using a synthetic oligonucleotide probe, (ii) a method for screening a transformant using a probe produced by PCR, (iii) a method for screening a transformant using an antibody against the polypeptide of the present invention, or (iv) a method for screening a transformant using a selective hybridization translation system, may be used.

In the method of the item (i) for screening a transformant using a synthetic oligonucleotide probe, the transformant containing the cDNA of interest may be selected, for example, by the following procedure.

An oligonucleotide which corresponds to the whole or a part of the polypeptide of the present invention is synthesized (in this case, it may be either a nucleotide sequence taking the codon usage into consideration or a plurality of nucleotide sequences as a combination of possible nucleotide sequences, and in the latter case, their numbers can be reduced by including inosine) and, using this oligonucleotide as a probe (labeled with ³²P or ³³P), hybridized with a nitrocellulose filter or a polyamide filter on which DNAs of the transformants are denatured and fixed, to screen and select resulting positive strains.

In the method of the item (ii) for screening a transformant using a probe produced by PCR, the transformant containing the cDNA of interest may be selected, for example, by the following procedure.

Oligonucleotides of a sense primer and an antisense primer corresponding to a part of the polypeptide of the present invention are synthesized, and a DNA fragment encoding the whole or a part of the polypeptide of interest is amplified by carrying out PCR using these primers in combination. As a template DNA used in this method, cDNA synthesized by a reverse transcription reaction from mRNA of cells capable of producing the polypeptide of the present invention, or genomic DNA, may be used. The resulting DNA fragment is labeled with ³²P or ³³P, and a transformant containing the cDNA of interest is selected by carrying out colony hybridization or a plaque hybridization using this fragment as a probe.

In the method of the item (iii) for screening a transformant using an antibody against the polypeptide of the present invention, the transformant containing the cDNA of interest may be selected, for example, by the following procedure.

Polypeptides are produced into a culture supernatant, inside the cells, or on the cell surface of transformants. A transformant containing the cDNA of interest is selected by detecting a strain producing the desired polypeptide using an antibody against the polypeptide of the present invention and a second antibody against the first antibody.

In the method of the item (iv) for screening a transformant using a selective hybridization translation system, the transformant containing the cDNA of interest may be selected, for example, by the following procedure.

First, cDNA obtained from each transformant is blotted on, for example, a nitrocellulose filter and hybridized with mRNA prepared from cells capable of producing the polypeptide of the present invention, and then the mRNA bound to the cDNA is dissociated and recovered. The recovered mRNA is translated into a polypeptide in an appropriate polypeptide translation system, for example, injection into Xenopus oocytes or a cell-free system such as a rabbit reticulocyte lysate or a wheat germ. A transformant containing the cDNA of interest is selected by detecting it with the use of an antibody against the polypeptide of the present invention.

A method for collecting the polynucleotide of the present invention from the resulting transformant of interest can be carried out in accordance with a known method (for example, Sambrook, J. et al., "Molecular Cloning-A Laboratory Manual", Cold Spring Harbor Laboratory, NY, 1989). For example, it may be carried out by separating a fraction corresponding to the plasmid DNA from cells and cutting out the cDNA region from the plasmid DNA.

In the chemical synthesis method of the item (3), the polynucleotide of the present invention may be produced, for example, by binding DNA fragments produced by a chemical synthesis method. Each DNA can be synthesized using a DNA synthesizer [for example, Oligo 1000M DNA Synthesizer (Beckman) or 394 DNA/RNA Synthesizer (Applied Biosystems)].

Further, the polynucleotide of the present invention may be produced by nucleic acid chemical synthesis in accordance with a conventional method such as a phosphite triester method (Hunkapiller, M. et al., Nature, 10, 105-111, 1984), based on the information on the polypeptide of the present invention. In this connection, codons for each amino acid are known and can be optionally selected and determined by the conventional method, for example, by taking a codon usage of each host to be used into consideration (Crantham, R. et al., Nucleic Acids Res., 9, r43-r74, 1981). Further, a partial modification of codons of these nucleotide sequences can be carried out in accordance with a conventional method, such as site directed mutagenesis which uses a primer comprised of a synthetic oligonucleotide coding for a desired modification (Mark, D. F. et al., Proc. Natl. Acad. Sci. USA, 81, 5662-5666, 1984).

Determination of the DNA sequences obtained by the above-mentioned methods can be carried out by, for example, a Maxam-Gilbert chemical modification method (Maxam, A. M. and Gilbert, W., "Methods in Enzymology", 65, 499-559, 1980) or a dideoxynucleotide chain termination method (Messing, J. and Vieira, J., Gene, 19, 269-276, 1982).

### [3] Expression vector and transformant of the present invention

An isolated polynucleotide of the present invention is re-integrated into an appropriate vector DNA and a eucaryotic or procaryotic host cell may be transfected by the resulting expression vector. Further, it is possible to express the polynucleotide in a desired host cell, by introducing an appropriate promoter and a sequence related to the gene expression into the vector.

The expression vector of the present invention is not particularly limited, so long as it comprises the polynucleotide of the present invention. As the expression vector, there may be mentioned, for example, an expression vector obtained by introducing the polynucleotide of the present invention into a known expression vector appropriately selected in accordance with a host cell to be used or a cell to be introduced. The expression vector of the present invention includes an expression vector for manufacturing the recombinant polypeptide of the present invention and an expression vector for producing the polypeptide of the present invention in a body by gene therapy.

The transformant of the present invention is not particularly limited, so long as it is transfected with the expression vector or the polynucleotide of the present invention and comprises the polynucleotide of the present invention. The transformant of the present invention may be, for example, a cell in which the polynucleotide is integrated into a chromosome of a host cell, or a cell containing the polynucleotide as an expression vector comprising polynucleotide. Further, the transformant of the present invention may be a cell expressing the polypeptide of the present invention, or a cell not expressing the polypeptide of the present invention. The transformant of the present invention may be obtained by, for example, transfecting a desired host cell with the expression vector of the present invention.

In the eucaryotic host cells, for example, cells of vertebrates, insects, and yeast are included. As the vertebral cell, there may be mentioned, for example, a simian COS cell (Gluzman, Y., Cell, 23, 175-182, 1981), a dihydrofolate reductase defective strain of a Chinese hamster ovary cell (CHO) (Urlaub, G. and Chasin, L. A., Proc. Natl. Acad. Sci. USA, 77, 4216-4220, 1980), a human fetal kidney derived HEK293 cell, a 293-EBNA cell (Invitrogen) obtained by introducing an EBNA-1 gene of Epstein Barr Virus into HEK293 cell, or a human 293T cell (DuBridge, R. B. et al., Mol. Cell. Biol., 7, 379-387, 1987).

As an expression vector for a vertebral cell, a vector containing a promoter positioned upstream of the gene to be expressed, an RNA splicing site, a polyadenylation site, a transcription termination sequence, and the like may be generally used. The vector may further contain a replication origin, if necessary. As the expression vector, there may be mentioned, for example, pSV2dhfr containing an SV40 early promoter (Subramani, S. et al., Mol. Cell. Biol., 1, 854-864, 1981), pEF-BOS containing a human elongation factor promoter (Mizushima, S. and Nagata, S., Nucleic Acids Res., 18,5322, 1990), or pCEP4 containing a cytomegalovirus promoter (Invitrogen).

When the COS cell is used as the host cell, a vector which has an SV40 replication origin, can perform an autonomous replication in the COS cell, and has a transcription promoter, a transcription termination signal, and an RNA splicing site, may be used as the expression vector. As the vector, there may be mentioned, for example, pME18S (Maruyama, K. and Takebe, Y., Med. Immunol., 20, 27-32, 1990), pEF-BOS (Mizushima, S. and Nagata, S., Nucleic Acids Res., 18, 5322, 1990), or pCDM8 (Seed, B., Nature, 329, 840-842, 1987).

The expression vector may be incorporated into COS cells by, for example, a DEAE-dextran method (Luthman, H. and Magnusson, G., Nucleic Acids Res., 11, 1295-1308, 1983), a calcium phosphate-DNA co-precipitation method (Graham, F. L. and van der Ed, A. J., Virology, 52, 456-457, 1973), a method using a commercially available transfection reagent (for example, FuGENE™6 Transfection Reagent; Boeringer Mannheim), or an electroporation method (Neumann, E. et al., EMBO J., 1, 841-845, 1982).

When the CHO cell is used as the host cell, a transfected cell capable of stably producing the polypeptide of the present invention can be obtained by carrying out co-transfection of an expression vector comprising the polynucleotide encoding the polypeptide of the present invention, together with a vector capable of expressing a neo gene which functions as a G418 resistance marker, such as pRSVneo (Sambrook, J. et al., "Molecular Cloning-A Laboratory Manual", Cold Spring Harbor Laboratory, NY, 1989) or pSV2-neo (Southern, P. J. and Berg, P., J. Mol. Appl. Genet., 1, 327-341,1982), and selecting a G418 resistant colony.

As a vector for a gene therapy, a vector generally used (for example, a retrovirus vector, an adenovirus vector, or Sendai virus vector) can be used.

The transformant of the present invention may be cultured in accordance with the conventional method, and the polypeptide of the present invention is produced inside the cells. As a medium to be used in the culturing, a medium commonly used in a desired host cell may be appropriately selected. In the case of the COS cell, for example, a medium such as an RPMI-1640 medium or a Dulbecco's modified Eagle's minimum essential medium (DMEM) may be used, by supplementing it with a serum component such as fetal bovine serum (FBS) if necessary. In the case of the 293-EBNA cell, a medium such as a Dulbecco's modified Eagle's minimum essential medium (DMEM) with a serum component such as fetal bovine serum (FBS) and G418 may be used.

The polypeptide of the present invention produced inside the transformant of the present invention by culturing the transformants may be separated and purified therefrom by various known separation techniques making use of the physical properties, chemical properties and the like of the polypeptide. More particularly, the polypeptide of the present invention may be purified by treating a cell extract containing the polypeptide of the present invention with a commonly used treatment, for example, a treatment with a protein precipitant, ultrafiltration, various liquid chromatography techniques such as molecular sieve chromatography (gel filtration), adsorption chromatography, ion exchange chromatography, affinity chromatography, or high performance liquid chromatography (HPLC), or dialysis, or a combination thereof.

When the polypeptide of the present invention is expressed as a fusion protein with a marker sequence in frame, identification of the expression of the polypeptide of the present invention, purification thereof, or the like may be easily carried out. As the marker sequence, there may be mentioned, for example, a FLAG tag, a hexa-histidine tag, a hemagglutinin tag, or a myc epitope. Further, by inserting a specific amino acid sequence recognized by a protease such as enterokinase, factor Xa, or thrombin between the marker sequence and the polypeptide of the present invention, the marker sequence may be removed by the protease.

### [4] Screening method of the present invention

It is possible to screen a substance which modifies (for example, inhibits or promotes) an SMGBP1 activity of the polypeptide according to the present invention, using the polypeptide of the present invention. Human SMGBP1 as the polypeptide of the present invention firmly binds to human SMG-1 that is essential to a process of mRNA surveillance, and thus, it is predicted that human SMGBP1 is closely involved in the modification of a kinase activity, intracellular localization, and the modification of an activity of a surveillance complex. Therefore, a substance modifying an SMGBP1 activity of the polypeptide of the present invention may modify nonsense mediated mRNA decay (NMD), for example, via a modification of an SMG-1 activity, and thus is useful as a candidate substance of an agent for treating and/or preventing a variety of diseases related to NMD. Further, the polypeptide of the present invention per se may be used as a screening tool for screening a substance modifying an SMGBP1 activity of the polypeptide of the present invention, or for screening an agent for treating and/or preventing various diseases related to NMD.

In this connection, NMD means a mechanism which recognizes and specifically degrades a nonsense mutation mRNA in which a codon in the inherent translational region of a gene is changed to a stop codon.

As the diseases related to NMD, there may be mentioned, for example, a disease due to mRNA having one or more premature translation termination codons (PTCs), which should be removed, but are not removed, or a disease caused by one or more PTCs generated by a nonsense mutation.

The disease caused by one or more PTCs generated by a nonsense mutation is not particularly limited, but there may be mentioned, for example, a genetic disease (for example, Duchenne type muscular dystrophy), cancer due to a somatic mutation, or the like. The important point is that, among all diseases caused by genome mutation, almost all diseases "due to one or more PTCs by a nonsense mutation" are included in such diseases.

One-quarter of the diseases caused by genome mutations are said to have the termination codon in the middle of a specific gene. The reasons for these diseases are that the protein consisting of the full-length polypeptide inherently encoded by the gene is not expressed, and that, due to the presence of the NMD mechanism, almost no protein fragments consisting of the N-terminal partial fragments of the full length polypeptide inherently encoded by the gene are expressed. However, even if there is a termination codon in the middle of the gene, and even if in the state of a protein fragment, there are cases of activity of the same extent as that of a full length polypeptide or the minimum necessary level, depending on the type of the gene or the position of the termination codon. In this case, if it were possible to inhibit the NMD mechanism, it would become possible to express a protein fragment having an effective activity, and thus it is theoretically predicted that at least part of a disease due to the presence of a termination codon in the middle of a specific gene, that is, a disease due to nonsense mutation of a specific gene, can be alleviated. However, no technique for a specific suppression of NMD has been found in the past.

Among the substances which are selected by the screening method of the present invention and modify an SMGBP1 activity of the polypeptide of the present invention, substances which can inhibit or suppress an SMG-1 activity via a modification of the SMGBP1 activity, may be expected to specifically inhibit or suppress NMD, and thus are useful as an active ingredient of a new type of agent for treatment and/or prevention, which can alleviate gene mutations for at least part of all sorts of diseases due to a nonsense mutation of a specific gene.

Alternatively, among the substances which are selected by the screening method of the present invention and modify an SMGBP1 activity of the polypeptide of the present invention, substances which can promote an SMG-1 activity via a modification of the SMGBP1 activity, may promote NMD, and thus are useful as an active ingredient of an agent for promoting NMD, or an agent for treating and/or preventing a disease due to mRNA having one or more PTCs, which should be removed, but are not removed.

Substances to be tested which may be applied to the screening method of the present invention are not particularly limited, but there may be mentioned, for example, various known compounds (including peptides) registered in chemical files, compounds obtained by combinatorial chemistry techniques (Terrett, N. K. et al., Tetrahedron, 51, 8135-8137, 1995) or conventional synthesis techniques, or random peptides prepared by employing a phage display method (Felici, F. et al., J. Mol. Biol., 222, 301-310, 1991) or the like. In addition, culture supernatants of microorganisms, natural components derived from plants or marine organisms, or animal tissue extracts may be used as the test substances for screening. Further, compounds (including peptides) obtained by chemically or biologically modifying compounds (including peptides) selected by the screening method of the present invention may be used.

The screening method of the present invention comprises the steps of:
(1) bringing the polypeptide of the present invention into contact with SMG-1 (for example, human SMG-1) and a substance to be tested under the conditions such that the polypeptide exhibits an SMGBP1 activity in the absence of the substance to be tested (hereinafter referred to as a contact step),and
(2) analyzing whether or not the polypeptide exhibits the SMGBP1 activity (hereinafter referred to as an analyzing step).

In the above contact step, the method for bringing the polypeptide of the present invention into contact with SMG-1 and a substance to be tested is not particularly limited, so long as these can be brought into contact with each other under the conditions such that the polypeptide of the present invention may exhibit an SMGBP1 activity (i.e., the polypeptide of the present invention may bind to SMG-1) in the absence of the test substance. As the contacting method, there may be mentioned, for example,
a method in which the polypeptide and SMG-1 are separately prepared by genetic engineering techniques, and the polypeptide, SMG-1, and the test substance are brought into contact with each other; or
a method in which a polynucleotide encoding the polypeptide is introduced into cells capable of producing SMG-1, the polypeptide and SMG-1 are produced in the cell in the presence of the test substance, and the polypeptide, SMG-1, and the test substance are brought into contact with each other.

In the above analyzing step, it is analyzed whether or not the polypeptide of the present invention exhibits an SMGBP1 activity. More particularly, for example, it is analyzed whether or not the complex of SMG-1 and the polypeptide of the present invention is formed.

In the analyzing step, when the complex of SMG-1 and the polypeptide of the present invention is not formed, it may be judged that the substance to be tested inhibits or suppresses an SMGBP1 activity of the polypeptide of the present invention.

In the analyzing step, as the method for analyzing the complex of SMG-1 and the polypeptide of the present invention, there may be mentioned, for example,
a method in which immunoprecipitation is performed using an antibody against either of two polypeptides, i.e., SMG-1 and the polypeptide of the present invention, (for example, anti-SMG-1 antibody), and the resulting immunoprecipitate is analyzed by Western blotting using an antibody against the other polypeptide (for example, antibody against the polypeptide of the present invention) to confirm the existence of the latter polypeptide [hereinafter referred to as the method using immunoprecipitation and Western blotting; for example, a method described in Example 4 (2)]; a method in which immunoprecipitation is performed using an antibody against either SMG-1 or the polypeptide of the present invention (for example, anti-SMG-1 antibody), and the resulting immunoprecipitate is electrophoresed and analyzed by an appropriate detection method (for example, silver staining) to confirm the existence of the other polypeptide (for example, the polypeptide of the present invention) [hereinafter referred to as the method using immunoprecipitation and silver staining; for example, a method described in Example 3(1)];
a method in which immunoprecipitation is performed using an anti-SMG-1 antibody, and the resulting immunoprecipitate is phosphorylated under the conditions such that phosphorylation by SMG-1 may be carried out to confirm the existence of the phosphorylated polypeptide of the present invention [hereinafter referred to as the method using immunoprecipitation and phosphorylation; for example, a method described in Example 1(2) to Example 1(4)]; or
a method in which chromatography is carried out to confirm whether or not the polypeptide of the present invention and SMG-1 exist in the same fraction [hereinafter referred to as the fraction method; for example, a method described in Example 2(2) to Example 2(3)].

When the above method using immunoprecipitation and Western blotting is used in the analyzing step, immunoprecipitation is carried out using an antibody against either of two polypeptides, i.e., SMG-1 and the polypeptide of the present invention, (for example, anti-SMG-1 antibody), and the resulting immunoprecipitate is analyzed by Western blotting using an antibody against the other polypeptide (for example, antibody against the polypeptide of the present invention). As a result of the Western blotting, when the latter polypeptide (for example, the polypeptide of the present invention) is detected, it may be judged that the complex of SMG-1 and the polypeptide of the present invention is formed (i.e. the polypeptide of the present invention exhibits an SMGBP1 activity). On the other hand, when the latter polypeptide (for example, the polypeptide of the present invention) is not detected, it may be judged that the complex of SMG-1 and the polypeptide of the present invention is not formed (i.e. the polypeptide of the present invention does not exhibit an SMGBP1 activity), and thus, it may be determined that the substance to be tested is a substance which inhibits or suppresses an SMGBP1 activity of the polypeptide of the present invention. Similarly, it may be determined whether or not a substance to be tested is a substance which promotes an SMGBP1 activity of the polypeptide of the present invention.

When the above method using immunoprecipitation and silver staining is used in the analyzing step, immunoprecipitation is performed using an antibody against either SMG-1 or the polypeptide of the present invention (for example, anti-SMG-1 antibody), and the resulting immunoprecipitate is electrophoresed and analyzed by an appropriate detection method (for example, silver staining) to confirm the existence of the other polypeptide (for example, the polypeptide of the present invention). When the latter polypeptide (for example, the polypeptide of the present invention) is detected, it may be judged that the complex of SMG-1 and the polypeptide of the present invention is formed (i.e. the polypeptide of the present invention exhibits an SMGBP1 activity). On the other hand, when the latter polypeptide (for example, the polypeptide of the present invention) is not detected, it may be judged that the complex of SMG-1 and the polypeptide of the present invention is not formed (i.e. the polypeptide of the present invention does not exhibit an SMGBP1 activity), and thus, it may be determined that the substance to be tested is a substance which inhibits or suppresses an SMGBP1 activity of the polypeptide of the present invention. Similarly, it may be determined whether or not a substance to be tested is a substance which promotes an SMGBP1 activity of the polypeptide of the present invention.

When the above method using immunoprecipitation and phosphorylation is used in the analyzing step, immunoprecipitation is performed using an anti-SMG-1 antibody, and the resulting immunoprecipitate is phosphorylated under the conditions such that phosphorylation by SMG-1 may be carried out to confirm the existence of the phosphorylated polypeptide of the present invention. When the polypeptide of the present invention is detected, it may be judged that the complex of SMG-1 and the polypeptide of the present invention is formed (i.e. the polypeptide of the present invention exhibits an SMGBP1 activity). On the other hand, when the polypeptide of the present invention is not detected, it may be judged that the complex of SMG-1 and the polypeptide of the present invention is not formed (i.e. the polypeptide of the present invention does not exhibit an SMGBP1 activity), and thus, it may be determined that the substance to be tested is a substance which inhibits or suppresses an SMGBP1 activity of the polypeptide of the present invention. Similarly, it may be determined whether or not a substance to be tested is a substance which promotes an SMGBP1 activity of the polypeptide of the present invention.

When the above fraction method is used in the analyzing step, chromatography is carried out to confirm whether or not the polypeptide of the present invention and SMG-1 exist in the same fraction. When SMG-1 and the polypeptide of the present invention exist in the same fraction, it may be judged that the complex of SMG-1 and the polypeptide of the present invention is formed (i.e. the polypeptide of the present invention exhibits an SMGBP1 activity). On the other hand, when the polypeptide of the present invention and SMG-1 do not exist in the same fraction, it may be judged that the complex of SMG-1 and the polypeptide of the present invention is not formed (i.e. the polypeptide of the present invention does not exhibit an SMGBP1 activity), and thus, it may be determined that the substance to be tested is a substance which inhibits or suppresses an SMGBP1 activity of the polypeptide of the present invention. Similarly, it may be determined that a substance to be tested is a substance which promotes an SMGBP1 activity of the polypeptide of the present invention.

### [5] Pharmaceutical composition of the present invention

Among the substances modifying an SMGBP1 activity, which may be selected by the screening method of the present invention, substances which suppress an SMG-1 activity via a modification of an SMGBP1 activity may suppress NMD, and thus are useful as a candidate substance of an agent for treating and/or preventing a disease caused by one or more PTCs generated by a nonsense mutation. The substance modifying an SMGBP1 activity can be administered to a subject (for example, an animal, preferably a mammal, particularly a human) in need of suppressing NMD or in need of treatment and/or prevention of a disease caused by one or more PTCs generated by a nonsense mutation, with or without, but preferably with, a pharmaceutically or veterinarily acceptable ordinary carrier or diluent, in an amount effective therefor.

On the other hand, among the substances modifying an SMGBP1 activity, which may be selected by the screening method of the present invention, substances which promote an SMG-1 activity of the polypeptide of the present invention via a modification of an SMGBP1 activity, can promote NMD, and thus are useful as a candidate substance of an agent for treating and/or preventing a disease due to mRNA having one or more PTCs, which should be removed, but are not removed. The substance modifying an SMGBP1 activity can be administered to a subject (for example, an animal, preferably a mammal, particularly a human) in need of promoting NMD or in need of treating and/or preventing a disease due to mRNA having one or more PTCs, which should be removed, but are not removed, with or without, but preferably with, a pharmaceutically or veterinarily acceptable ordinary carrier or diluent, in an amount effective therefor.

The formulation of the pharmaceutical composition of the present invention is not particularly limited to, but may be, for example, oral medicines, such as powders, fine particles, granules, tablets, capsules, suspensions, emulsions, syrups, extracts or pills, or parenteral medicines, such as injections, liquids for external use, ointments, suppositories, creams for topical application, or eye lotions.

The oral medicines may be prepared by an ordinary method using, for example, fillers, binders, disintegrating agents, surfactants, lubricants, flowability-enhancers, diluting agents, preservatives, coloring agents, perfumes, tasting agents, stabilizers, humectants, antiseptics, antioxidants or the like, such as gelatin, sodium alginate, starch, corn starch, saccharose, lactose, glucose, mannitol, carboxylmethylcellulose, dextrin, polyvinyl pyrrolidone, crystalline cellulose, soybean lecithin, sucrose, fatty acid esters, talc, magnesium stearate, polyethylene glycol, magnesium silicate, silicic anhydride, or synthetic aluminum silicate.

The parenteral administration may be, for example, an injection such as a subcutaneous or intravenous injection, or a per rectum administration. Of the parenteral formulations, an injection is preferably used.

When the injections are prepared, for example, watersoluble solvents, such as physiological saline or Ringer's solution, water-insoluble solvents, such as plant oil or fatty acid ester, agents for rendering isotonic, such as glucose or sodium chloride, solubilizing agents, stabilizing agents, antiseptics, suspending agents, or emulsifying agents may be optionally used, in addition to the active ingredient.

The pharmaceutical composition of the present invention may be administered in the form of a sustained release preparation using sustained release polymers. For example, the pharmaceutical composition of the present invention may be incorporated to a pellet made of ethylenevinyl acetate polymers, and the pellet may be surgically implanted in a tissue to be treated.

The pharmaceutical composition of the present invention may contain the active ingredient in an amount of, but is by no means limited to, 0.01 to 99% by weight, preferably 0.1 to 80% by weight.

A dose of the pharmaceutical composition of the present invention is not particularly limited, but may be determined dependent upon, for example, the kind of the active ingredient, the kind of disease, the age, sex, body weight, or symptoms of the subject, a method of administration, or the like. The pharmaceutical composition of the present invention may be orally or parenterally administered.

The pharmaceutical composition of the present invention may be administered as a medicament or in various forms, for example, eatable or drinkable products, such as functional foods or health foods, or feeds.

### [6] Antibody of the present invention

An antibody, such as a polyclonal antibody or a monoclonal antibody, which reacts with the polypeptide of the present invention may be obtained by directly administering the polypeptide of the present invention or a fragment thereof to various animals. Alternatively, it may be obtained by a DNA vaccine method (Raz, E. et al., Proc. Natl. Acad. Sci. USA, 91, 9519-9523, 1994; or Donnelly, J. J. et al., J. Infect. Dis., 173, 314-320, 1996), using a plasmid into which a polynucleotide encoding the polypeptide of the present invention is inserted.

The polyclonal antibody may be produced from a serum or eggs of an animal such as a rabbit, a rat, a goat, or a chicken, in which the animal is immunized and sensitized by the polypeptide of the present invention or a fragment thereof emulsified in an appropriate adjuvant (for example, Freund's complete adjuvant) by intraperitoneal, subcutaneous, or intravenous administration. The polyclonal antibody may be separated and purified from the resulting serum or eggs in accordance with conventional methods for polypeptide isolation and purification. Examples of the separation and purification methods include, for example, centrifugal separation, dialysis, salting-out with ammonium sulfate, or a chromatographic technique using such as DEAE-cellulose, hydroxyapatite, protein A agarose, and the like.

The monoclonal antibody may be easily produced by those skilled in the art, according to, for example, a cell fusion method of Kohler and Milstein (Kohler, G. and Milstein, C., Nature, 256, 495-497, 1975).

A mouse is immunized intraperitoneally, subcutaneously, or intravenously several times at an interval of a few weeks by a repeated inoculation of emulsions in which the polypeptide of the present invention or a fragment thereof is emulsified into a suitable adjuvant such as Freund's complete adjuvant. Spleen cells are removed after the final immunization, and then fused with myeloma cells to prepare hybridomas.

As a myeloma cell for obtaining a hybridoma, a myeloma cell having a marker such as a deficiency in hypoxanthine-guanine phosphoribosyltransferase or thymidine kinase (for example, mouse myeloma cell line P3X63Ag8.U1) may be used. As a fusing agent, polyethylene glycol may be used. As a medium for preparation of hybridomas, for example, a commonly used medium such as an Eagle's minimum essential medium, a Dulbecco's modified minimum essential medium, or an RPMI-1640 medium may be used by adding properly 10 to 30% of a fetal bovine serum. The fused strains may be selected by a HAT selection method. A culture supernatant of the hybridomas is screened by a well-known method such as an ELISA method or an immunohistological method, to select hybridoma clones secreting the antibody of interest. The monoclonality of the selected hybridoma is guaranteed by repeating subcloning by a limiting dilution method. Antibodies in an amount which may be purified are produced by culturing the resulting hybridomas in a medium for 2 to 4 days, or in the peritoneal cavity of a pristane-pretreated BALB/c strain mouse for 10 to 20 days.

The resulting monoclonal antibodies in the culture supernatant or the ascites may be separated and purified by conventional polypeptide isolation and purification methods. Examples of the separation and purification methods include, for example, centrifugal separation, dialysis, salting-out with ammonium sulfate, or chromatographic technique using such as DEAE-cellulose, hydroxyapatite, protein A agarose, and the like.

Further, the monoclonal antibodies or the antibody fragments containing a part thereof may be produced by inserting the whole or a part of a gene encoding the monoclonal antibody into an expression vector and introducing the resulting expression vector into appropriate host cells (such as E. coli, yeast, or animal cells).

Antibody fragments comprising an active part of the antibody such as F(ab')₂, Fab, Fab', or Fv may be obtained by a conventional method, for example, by digesting the separated and purified antibodies (including polyclonal antibodies and monoclonal antibodies) with a protease such as pepsin or papain, and separating and purifying the resulting fragments by standard polypeptide isolation and purification methods.

Further, an antibody which reacts to the polypeptide of the present invention may be obtained in a form of single chain Fv or Fab in accordance with a method of Clackson et al. or a method of Zebedee et al. (Clackson, T. et al., Nature, 352, 624-628, 1991; or Zebedee, S. et al., Proc. Natl. Acad. Sci. USA, 89, 3175-3179, 1992). Furthermore, a humanized antibody may be obtained by immunizing a transgenic mouse in which mouse antibody genes are substituted with human antibody genes (Lonberg, N. et al., Nature, 368, 856-859, 1994).

### [7] Knockout non-human animal and cell of the present invention

The knockout non-human animal of the present invention is not particularly limited, so long as the expression of the gene encoding the polypeptide of the present invention is partially or completely suppressed. It may be prepared by a method known per se.

For example, by using a recombinant vector containing the polynucleotide of the present invention, and embryonic stem cells of the target non-human animal, such as cow, sheep, goat, pig, horse, mouse, or chicken, the gene encoding the polypeptide of the present invention on the chromosomes thereof is deactivated by a known homologous recombinant technique [for example, Nature, 326, 6110, 295 (1987); or Cell, 51, 3, 503 (1987)], or is replaced with any sequence [for example, Nature, 350, 6315, 243 (1991)], to prepare mutant clones. By using the mutant clones of the embryonic stem cells, a chimeric individual consisting of embryonic stem cell clones and normal cells may be prepared by a technique such as an aggregation chimera method or an injection chimera method into blastocyst in fertilized eggs of the animal. By combining the chimera individual and a normal individual, it is possible to obtain an individual having any mutation in the gene encoding the polypeptide of the present invention located on the chromosomes of cells of the entire body. By further combining the individuals, it is possible to obtain, from homozygous individuals with the mutation in both homologous chromosomes, a knockout non-human animal as an individual in which the expression of the gene encoding the polypeptide of the present invention is partially or completely suppressed.

Further, the cell of the present invention in which the expression of the gene encoding the polypeptide of the present invention is partially or completely suppressed, may be obtained by the above knockout non-human animal.

Furthermore, for the desired cell (for example, a human fatal kidney derived HEK293 cell, a 293-EBNA cell obtained by introducing the EBNA-1 gene of Epstein Barr Virus into the HEK293 cell, or a human 293T cell), the expression of intracellular endogenous SMGBP1 can be suppressed by an RNA interference method (for example, Nature, 411, 494-498, 2002). The present invention also includes the cells.

Further, by introducing mutation into any site of a gene encoding the polypeptide of the present invention on the chromosome, it is also possible to produce a knockout non-human animal. For example, by substituting, deleting, and/or inserting one or more bases with respect to a translation region of the gene encoding the polypeptide of the present invention on the chromosome, it is possible to modify the activity of the gene product.

Further, by introducing a similar mutation in the expression control region, it is also possible to modify, for example, the degree of expression, period of expression, and/or a tissue specificity. Further, by the combination with the Cre-loxP system, it is possible to control, for example, the period of expression, location of expression, and/or the amount of expression, more directly. As such examples, an example in which, using a promoter expressed at a specific region of the brain, the target gene was deleted at only the specific region [Cell, 87, 7, 1317, 1996)], or an example in which, using an adenovirus expressing Cre, the target gene was deleted from the specific organ at the desired period [Science, 278, 5335 (1997)] are known.

Therefore, even for a gene encoding the polypeptide of the present invention on the chromosome, it is possible to control expression at any period or tissue as described above. Further, it is possible to prepare a knockout non-human animal having any insertion, deletion, and/or substitution at the translation region or expression control region. A knockout non-human animal can induce the symptoms of various diseases derived from the polypeptide of the present invention at any period, to any degree, and/or at any location. As described above, the knockout non-human animal of the present invention becomes an extremely useful animal model in the treatment or prevention of various diseases derived from the polypeptide of the present invention.

Further, the knockout non-human animal of the present invention can be used to establish a model animal of a disease due to a gene different from the gene encoding the polypeptide of the present invention. For example, one of the knockout non-human animals of the present invention, that is, an SMGBP1 knockout mouse, and various lines of (apparent) normal mice can be combined. When the normal mouse contains a mutant gene having one or more PTCs, in a mouse obtained by the combination (for example, a homozygous individual having the SMGBP1 mutation in both of the homologous chromosomes or a heterozygous individual having the SMGBP1 mutation in one of the homologous chromosomes), NMD activity is expected to be affected, for example, when NMD is suppressed, and thus the mRNA derived from the mutant gene increases. As a result, for a certain mutant gene, sometimes hidden symptoms surface and some sort of disease appears. It is possible to establish a new disease model mouse.

### EXAMPLES

The present invention now will be further illustrated by, but is by no means limited to, the following Examples.

### Example 1: Detection of hSMG-1 binding protein phosphorylated dependently on the enzymatic activity of human SMG-1 (hSMG-1)

### (1) Cultivation and collection of cells and preparation of cell extract

Human HeLa cells (ATCC: CCL-2) were cultured to 10⁷ cells in accordance with a conventional method. Cells in a culture dish were washed with ice-cold phosphate-buffered saline (PBS), scraped with a cell scraper, and collected by centrifugation. After removing PBS, 3 mL of an F-lysis solution [20 mmol/L Tris-HCl (pH7.5), 0.25 mol/L sucrose, 20 mmol/L β-mercaptoethanol, 1.2 mmol/L EGTA, 1 mmol/L sodium orthovanadate, 1 mmol/L sodium fluoride, 1 mmol/L sodium pyrophosphate, 150 mmol/L sodium chloride, 1% Triton X-100, and 0.5% Nonidet P40] supplemented with protease inhibitors [1 mmol/L phenylmethylsulfonyl fluoride (PMSF), 10 µg/mL aprotinin, and 10 µg/mL leupeptin] was added and thoroughly stirred. Further, the whole was sonicated to completely disrupt the cells. The resultant was centrifuged at 15000 r.p.m. for 15 minutes to collect the supernatant as a cell extract.

### (2) Immunoprecipitation

In this example, antiserum C [Yamashita, Akio, et al, "GENES & DEVELOPMENT", (USA), 2001, 15, p. 2215-2228] was used as an anti-hSMG-1 serum. Antiserum C was obtained by using as an immunogen a C-terminal fragment [corresponding to the sequence consisting of the 3076th to 3542nd amino acid residues in the amino acid sequence of hSMG-1 (3657 amino acids)] of hSMG-1, which was expressed in E. coli as a protein (molecular weight = approximately 70 kDa) fused to glutathione S-transferase (GST).

To 25 µL of protein A Sepharose particles (Amersham Pharmacia), on which 10 µL of the anti-hSMG-1 serum (antiserum C) or the preimmune serum thereof (pre) was previously adsorbed, 1 mL of the cell extract (centrifugation supernatant) obtained in Example 1(1) was added and stirred at 4°C for 2 hours. After the whole was centrifuged to remove the supernatant, washing solution A (the F-lysis solution supplemented with 0.25 mol/L lithium chloride) was newly added and stirred gently for a short time, and the supernatant was removed by recentrifugation. The washing step was further repeated six times to remove non-specific adsorbates.

### (3) Phosphorylation

The resulting protein A Sepharose particles were washed with a 1×kinase reaction solution [10 mmol/L Hepes (pH7.5), 50 mmol/L sodium chloride, 50 mmol/L β-glycerophosphoric acid, and 1 mmol/L dithiothreitol (DTT)] once. To the particles, 25 µL of a 2×kinase reaction solution supplemented with divalent metal ion(s) (20 mmol/L MgCl₂ or 20 mmol/L MnCl₂, or both) and/or 1 µmol/L wortmannin [an inhibitor against phosphatidyl inositol 3-kinase (PI3-kinase)-related protein kinase] was added and stirred a little, and then the whole was allowed to stand on ice for 20 minutes. Further, 25 mL of starting solution (5 µCi [γ-³²P]ATP and 10 µmol/L ATP) was added and stirred a little to carry out a reaction at 30°C for 15 minutes. The reaction was stopped by adding 50 µL of a 2×SDS sample buffer [200 mmol/L Tris-HCl (pH6.8), 4% sodium dodecyl sulfate (SDS), 12% β-mercaptoethanol, 20% glycerol, and 0.02% bromophenol blue], and the whole was incubated at 100°C for 4 minutes to denature proteins. Each supernatant thereof was used in the following analysis.

### (4) Analysis of phosphorylated proteins

To analyze phosphorylated proteins having various molecular weights from a high molecular weight to a low molecular weight with a high resolution, samples to be analyzed were electrophoresed by SDS-polyacrylamid gel electrophoresis (SDS-PAGE) using two different gels having a gel concentration of 6% or 12.5%. After the electrophoresis, the gels were dried, and phosphorylated bands were visualized by autoradiography in accordance with conventional methods.

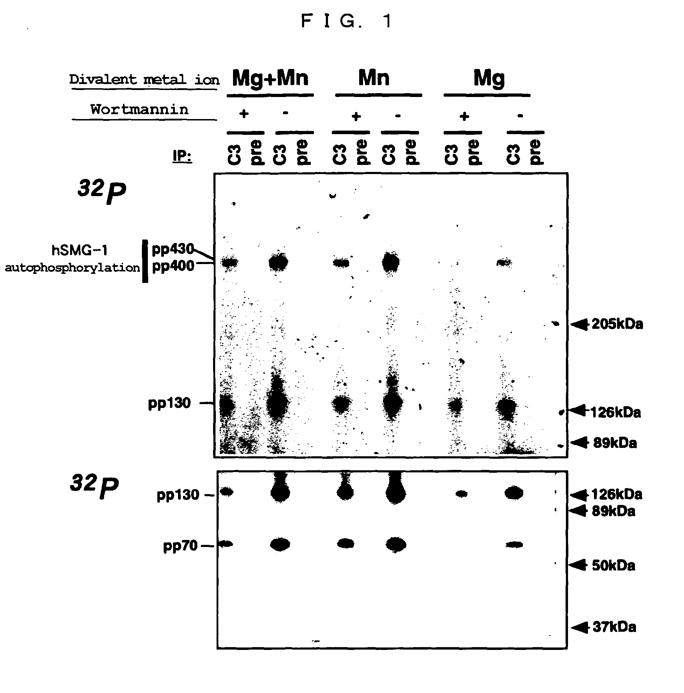

The results are shown in Figure 1. In Figure 1, the symbol "³²P" indicates the results obtained by autoradiography. The symbols "+" and "-" indicate the results obtained by performing phosphorylation in the presence of wortmannin, and in the absence thereof, respectively. The symbol "IP" means immunoprecipitation, and the symbols "C3" and "pre" indicate the results obtained by immunoprecipitation using antiserum C and the preimmune serum thereof, respectively. Each molecular weight (for example, "205kDa") and each arrow shown at the right of the electrophoretic pattern show the molecular weight of each protein used as molecular markers, and the electrophoresed position thereof, respectively. The symbols "pp430", "pp400", "pp130", and "pp70" shown at the left of the electrophoretic pattern indicate that each protein having a molecular weight of the number in each symbol is phosphorylated.

As shown in Figure 1, phosphorylated bands were detected at the positions of 400 kDa and 430 kDa. It is supported, from the facts described below, that these bands are autophosphorylated hSMG-1. First, the positions of the two phosphorylated bands accorded with the molecular weights of hSMG-1 detected by Western blotting. Further, the two bands were not detected from the immunoprecipitate precipitated with the preimmune serum. Furthermore, the phosphorylated bands were remarkably decreased in the presence of wortmannin, which was an inhibitor against PI3-kinase-related protein kinases including hSMG-1. Furthermore, the 430 kDa protein and the 400 kDa protein were phosphorylated more strongly when Mn was added, in comparison with the addition of Mg. In this connection, it is known that PI3-kinase-related protein kinases require a divalent metal ion to show the activities (including the autophosphorylation activity) thereof, and prefer Mg to Mn as the divalent metal ion.

In this example, two proteins, that is, a protein having a molecular weight of 130 kDa (p130) and a protein having a molecular weight of 70 kDa (p70), were detected as phosphorylated proteins. The patterns of phosphorylation thereof accorded exactly with those of autophosphorylation of hSMG-1. The results indicate the possibility that the two proteins are binding proteins which may be phosphorylated by the kinase activity of hSMG-1.

To support the possibility, the above immunoprecipitation and phosphorylation were repeated, except for the use of another anti-hSMG-1 serum, antiserum L, instead of antiserum C. In this connection, antiserum L was obtained by using as an immunogen an N-terminal fragment [corresponding to the sequence consisting of the 864th to 1059th amino acid residues in the amino acid sequence of hSMG-1 (3657 amino acids)] of hSMG-1, which was expressed in E. coli as a protein (molecular weight = approximately 50 kDa) fused to GST.

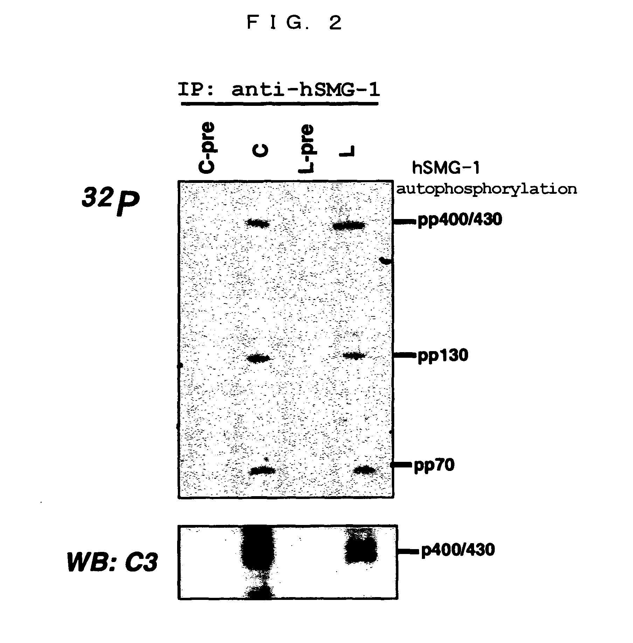

The results are shown in Figure 2. In Figure 2, the symbol "³²P" indicates the results obtained by autoradiography, and the symbol "WB:C3" indicates the results obtained by Western blotting using antiserum C. The symbols "C-pre", "C", "L-pre", and "L" indicate the results obtained by immunoprecipitation using the preimmune serum of antiserum C, antiserum C, the preimmune serum of antiserum L, and antiserum L, respectively.

When not only antiserum C, but also antiserum L, obtained by using a different region of hSMG-1 as an immunogen, was used, two proteins of 130 kDa and 70 kDa were detected as phosphorylated proteins. The results increase the above possibility, and exclude the possibility that one or more proteins nonspecifically immunoprecipitated by antiserum C is accidentally phosphorylated by hSMG-1.

### Example 2: Fractionation of HeLa cell extract by liquid column chromatography and phosphorylation in hSMG-1 immunocomplex

### (1) Preparation of cell extract

The following procedures were carried out at 4°C or less.

HeLa cells were cultured to 3 × 10⁹ cells in accordance with a conventional method, and collected in PBS by the method described in Example 1(1). To the cells, after removing the supernatant by centrifugation, 35 mL of buffer TG [20 mmol/L Tris-HCl (pH7.5), 2 mmol/L EDTA, 2 mmol/L DTT, 1 mmol/L sodium orthovanadate, and 10% glycerol] supplemented with 0.25% Triton X-100 and protease inhibitors (0.1 mmol/L PMSF, 10 µg/mL aprotinin, and 10 µg/mL leupeptin) was added and stirred gently. The cell suspension was transferred to a Potter type homogenizer, and disrupted by moving a pestle made of polytetrafluoroethylene for 50 strokes in the homogenizer, to extract proteins. The whole was ultracentrifuged at 100000xg for 60 minutes to obtain a cell extract. The cell extract was filtered through a membrane filter (0.22 µm pore size; Millipore) to prepare a sample to be analyzed.

### (2) Liquid column chromatography

Four anion exchange columns (HiTrapQ; resin volume = 5 mL; Pharmacia) were joined, connected to a liquid column chromatography system (FPLC; Pharmacia), and equilibrated with buffer A (Buffer TG supplemented with 0.1% Triton X-100). The sample to be analyzed prepared in Example 2(1) was applied to the column, and the column was thoroughly washed with buffer A. Proteins were eluted from the resins under conditions such that the ratio of buffer B (buffer A supplemented with 1 mol/L sodium chloride) in buffer A was linearly increased from 0% to 60% for 85 minutes (flow rate = 1.5 mL/min.). A fraction collector attached to the FPLC system was used to fractionate eluates at a rate of 2 min./fraction (approximately 3 mL) continuously. Further, the degrees of eluted proteins were monitored at an absorbance of 280 nm.

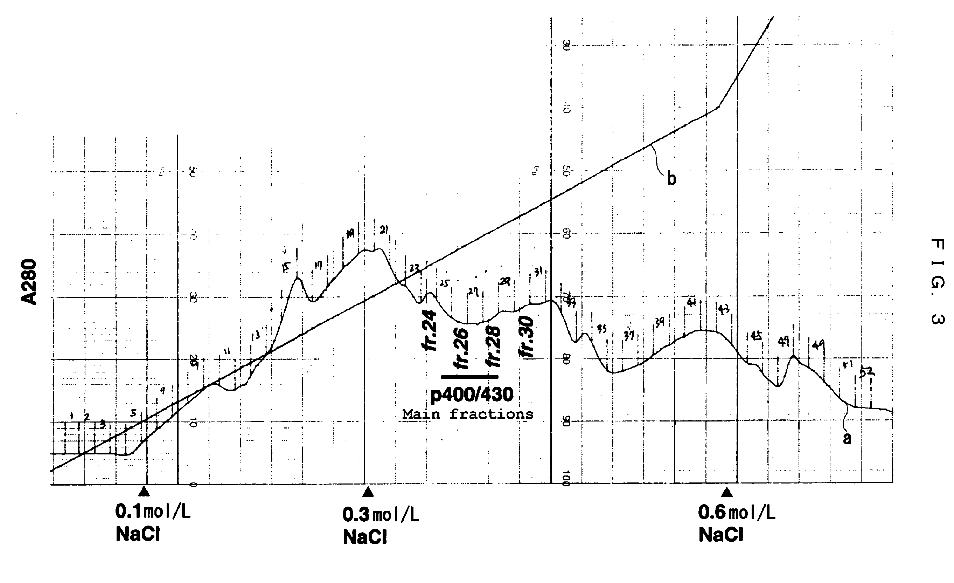

The results are shown in Figure 3. In Figure 3, curve a indicates the absorbance at 280 nm (A280), i.e., relative concentration of proteins, and line b indicates a concentration gradient of sodium chloride. The symbol "fr." means a fraction. Each number following the symbol "fr.", and each hand-written number described along the curve a is a fraction number.

### (3) Immunoprecipitation of hSMG-1 from each fraction and phosphorylation

Each fraction (equivalent of 10 µL) was analyzed by Western blotting using anti-hSMG-1 antibody C or anti-hSMG-1 antibody P.

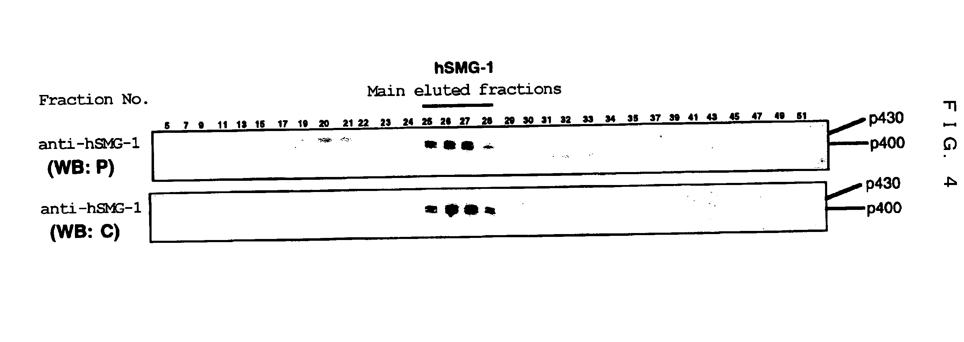

The results are shown in Figure 4. The symbols "WB:P" and "WB:C" indicate the results obtained by Western blotting using antiserum P and antiserum C, respectively.

It was found that concentrated hSMG-1 was eluted in fractions 25 to 28.

Each fraction (600 µL) was used to carry out immunoprecipitation and phosphorylation by the methods described in Example 1(2) and Example 1(3), except that an amount of the antibody (antiserum C) used in immunoprecipitation was 1 µL/fraction; and that the 2×kinase reaction solution used in phosphorylation did not contain wortmannin, but contained 10 mmol/L MnCl₂ as the divalent metal ion, and further contained 2 µg of a GST-p53(1-58) protein as an exogenous substrate. In this connection, the GST-p53(1-58) protein was a fusion protein of GST and a fragment consisting of the 1st to 58th amino acids in protein p53 (amino acid residues = 393). It is known that the 15th Serine residue (Ser15) and the 37th Serine residue (Ser37) in protein p53 locate in a sequence which accords with a phosphorylated consensus motif of hSMG-1, and are phosphorylated by hSMG-1 [Yamashita, Akio, et al, "GENES & DEVELOPMENT", (USA), 2001, 15, p. 2215-2228].

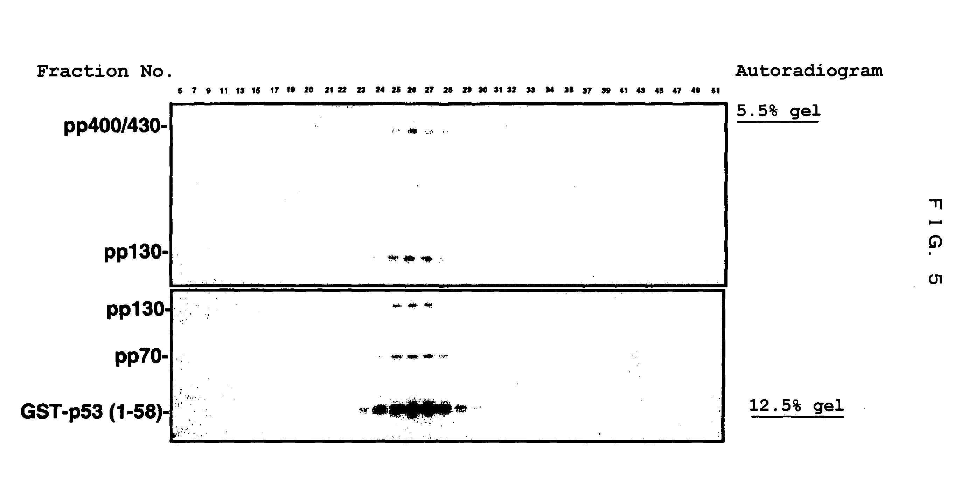

After phosphorylation, the reaction solutions were analyzed by SDS-PAGE and autoradiography. The results are shown in Figure 5. After the column fractionation was carried out, the autophosphorylation activity and the activity against exogenous-substrate of hSMG-1 were not separated from p130 and p70. The results strongly suggest that hSMG-1 always behaves together with p130 and p70.

Next, fractions 20 to 31 were used to carry out immunoprecipitation and phosphorylation by the methods described in Example 1(2) and Example 1(3), except that the 2×kinase reaction solution used in phosphorylation contained 2 µg of the GST-p53(1-58) protein as an exogenous substrate; the starting solution contained 1 mmol/L ATP, but did not contain radioactive ATP; and the reaction was carried out for 1 hour.

The states of the phosphorylated GST-p53(1-58) protein were analyzed by SDS-PAGE, and Western blotting using an antibody against phosphorylated Ser15 in protein p53 (anti P-Ser15 antibody; Shieh, S.Y. et al., Cell, 91, 325-334, 1997) or an antibody against phosphorylated Ser37 in protein p53 (anti P-Ser37 antibody; Kitagawa, M. et al., EMBO J., 15, 7060-7069, 1996).

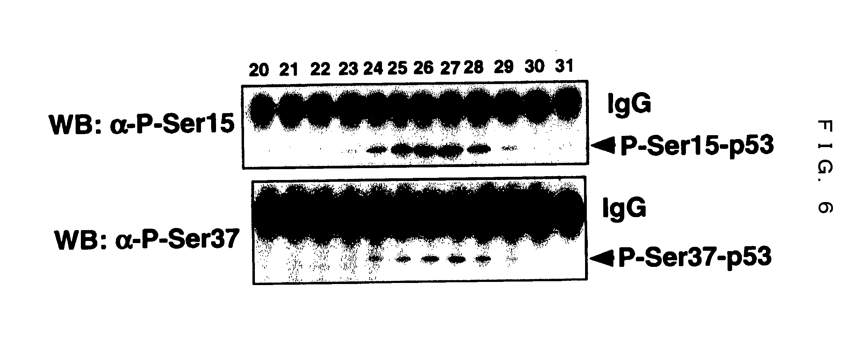

The results are shown in Figure 6. The symbols "WB:α-P-Ser15" and "WB:α-P-Ser37" indicate the results obtained by Western blotting using the anti P-Ser15 antibody and the anti P-Ser37 antibody, respectively. The symbols "P-Ser15-p53" and "P-Ser37-p53" mean the GST-p53(1-58) proteins phosphorylated at Ser15 and Ser37, respectively.

### Example 3: Purification of hSMG-1 complex by antibody column and analysis thereof

### (1) Immunoprecipitation

The HeLa cell extract was prepared by the method described in Example 1(1). To the cell extract, 3 mL (volume) of protein A Sepharose (Amersham-Pharmacia) was added, and stirred at 4°C for 30 minutes. The whole was centrifuged to obtain the supernatant. To 100 µL of protein A Sepharose, on which 25 µg of anti-hSMG-1 IgG (antibody N) purified from antiserum N by a conventional method, or 25 µg of normal rabbit IgG (Santa Cruz), was previously adsorbed, half of the supernatant was added and stirred at 4°C for 2 hours. In this connection, antiserum N was obtained by using as an immunogen an N-terminal fragment [corresponding to the sequence consisting of the 1st to 106th amino acid residues in the amino acid sequence of hSMG-1 (3657 amino acids)] of hSMG-1, which was expressed in E. coli as a protein fused to GST.

After the whole was centrifuged to remove the supernatant, the F-lysis solution was newly added and stirred for a short time, and the supernatant was removed by recentrifugation. The step was repeated six times. To the collected protein A Sepharose particles, 100 µL of a 2×SDS-sample buffer was added, and boiled at 100°C for 4 minutes. The whole was centrifuged to obtain the supernatant as samples to be analyzed.

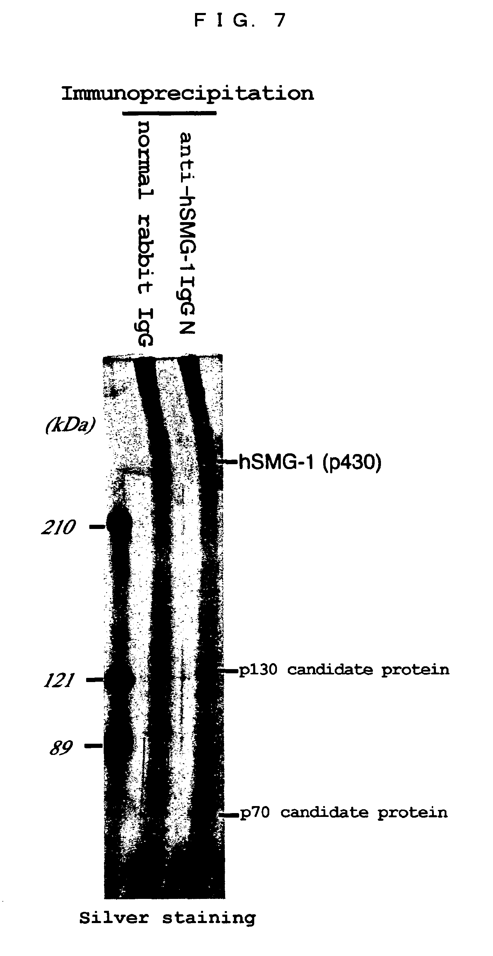

In accordance with conventional methods, 1/20 of the samples were analyzed by SDS-PAGE and silver staining. The results are shown in Figure 7. In the immunoprecipitate by the anti hSMG-1 IgG antibody, a band was detected at the position of approximately 130 kDa. The band was not detected in the immunoprecipitate by the normal rabbit IgG. In addition, a band, which was considered to be immunoprecipitated hSMG-1, was detected at the position of approximately 430 kDa.

### (2) Mass spectrometry

In accordance with conventional methods, 1/4 of the samples to be analyzed were subjected to SDS-PAGE, and transferred to a polyvinylidene difluoride (PVDF) membrane (Immobilon C; Millipore). After the transfer, the membrane was immersed in a Coomassie brilliant blue (CBB) solution for a short time, washed in water, and dried, to observe transferred and stained proteins. A band considered to be 430 kDa and a band considered to be the phosphorylated protein of 130 kDa were detected.

The stained protein of 130 kDa (p130) was excised from the membrane with a knife. In accordance with a known method [for example, Akihiko Iwamatsu, Experimental medicine (Jikken Igaku), Vol. 17, No. 8, 1999, p. 1351-1355, "Close-up in experimental methods -Proteome analysis by mass spectrometer-"], the protein was thoroughly digested with protease Lys-C (lysylendopeptidase) on the membrane, and the digested fragments were analyzed by mass spectrometry using MALDI-TOF/MS (matrix assisted laser desorption ionization time-of-flight mass spectrometry).

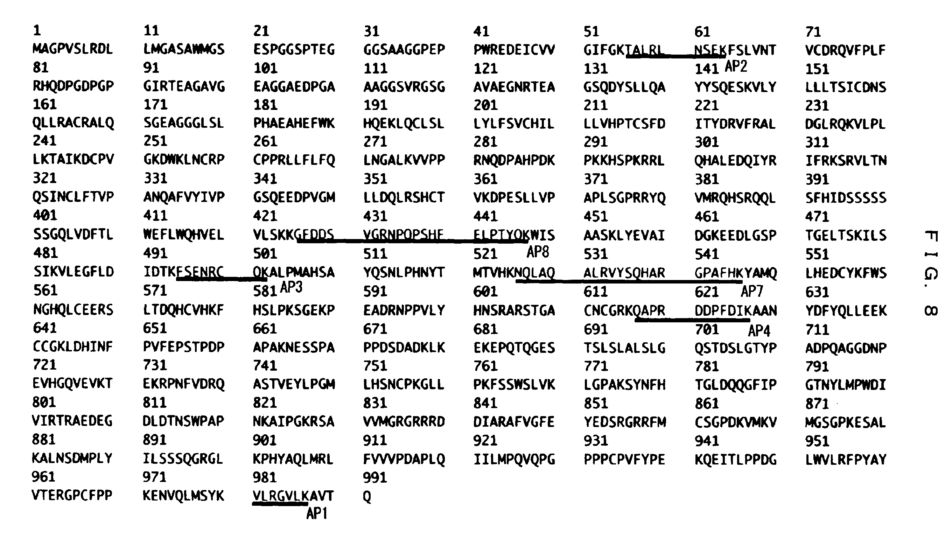

As a result, each mass of eight Lys-C-digested fragments (AP1 to AP8) shown in Table 1 was determined. A search through known databases in the primary structure of proteins was carried out. Among the eight masses, six masses of protein fragments (AP1, AP2, AP3, AP4, AP5, and AP8) accorded with those of Lys-C-digested fragments deduced from a translated product of cDNA clone AK026858.1 registered in known databases. In Figure 8, the primary structure (the amino acid sequence of SEQ ID NO: 2) of the translated product of AK026858.1, and the positions of sequences corresponding to AP1, AP2, AP3, AP4, AP5, and AP8 are shown. From the results, it was supposed that p130 was the translated product of AK026858.1, and an hSMG-1 binding protein. The translated product of AK026858.1 contains no region homologous to known functional motifs. However, genes which may encode proteins having a high homology with the translated product of AK026858.1 exist in Drosophila (CG6729; homology = 41%) and Caenorhabditis elegans (K04B12.3; homology = 41%), and thus these proteins are considered to evolutionarily conserved proteins. In this connection, each homology described above is a value determined by BLAST (Basic local alignment search tool; Altschul, S. F. et al., J.Mol.Biol., 215, 403-410, 1990).

**Table 1**

| Peptide | Mass (measured) | Mass (calculated) | Error | Sequence |
|---|---|---|---|---|
| AP1 | 784.54 | 784.54 | 0.00 | SEQ ID NO: 3 |
| AP2 | 1031.61 | 1031.58 | 0.03 | SEQ ID NO: 4 |
| AP3 | 1069.55 | 1069.47 | 0.08 | SEQ ID NO: 5 |
| AP4 | 1301.71 | 1301.65 | 0.06 | SEQ ID NO: 6 |
| AP5 | 1599.86 | - | - | - |
| AP6 | 2196.10 | - | - | - |
| AP7 | 2392.28 | 2392.27 | 0.01 | SEQ ID NO: 7 |
| AP8 | 2519.26 | 2519.20 | 0.06 | SEQ ID NO: 8 |

### Example 4: Preparation of anti-p130 antibody and analysis of expressed protein

### (1) Preparation of antibody

The AK026858.1 DNA had been identified in the Full-length human cDNA sequencing project by Dr. Sugano et al. in the Institute of Medical Science, the University of Tokyo, and the plasmid used in this example, in which the AK026858.1 DNA was inserted into pME18S, was provided from Dr. Sugano. To confirm that the translated product of AK026858.1 is an hSMG-1 binding protein, a specific antibody thereagainst was prepared.

The plasmid containing AK026858.1 was digested with restriction enzymes SmaI and MscI to obtain a DNA fragment of 446 bp (corresponding to the amino acid sequence consisting of the 563rd to 710th amino acids in the amino acid sequence of SEQ ID NO: 2). The DNA fragment was inserted at the SmaI site of E. coli expression vector pGEX-6P-1 (Amersham Pharmacia), and the resulting recombinant plasmid was introduced into E. coli DH5. E. coli strains containing the recombinant plasmid were cultured, and the p130 protein fragment was purified as a protein fused to GST, in accordance with a conventional method. In accordance with a conventional method, rabbits (New Zealand White) were immunized with the purified fusion protein as an immunogen to obtain an antiserum. Further, a specific antibody (anti AK026858.1-translated-product antibody) was separated from the antiserum by a conventional method (affinity purification method) using the immunogen protein.

### (2) Expression of p130 in HeLa cells and confirmation of hSMG-1 binding activity by immunoprecipitation

The HeLa cell extract was prepared by the method described in Example 1(1), and immunoprecipitation using the normal rabbit serum or the anti-hSMG-1 antiserums (antiserum N, antiserum L, or antiserum C) was carried by the method described in Example 3(1). The cell extract and immunocomplex were subjected to SDS-PAGE, and analyzed by Western blotting using the anti AK026858.1-translated-product antibody prepared in Example 4(1), in accordance with conventional methods.

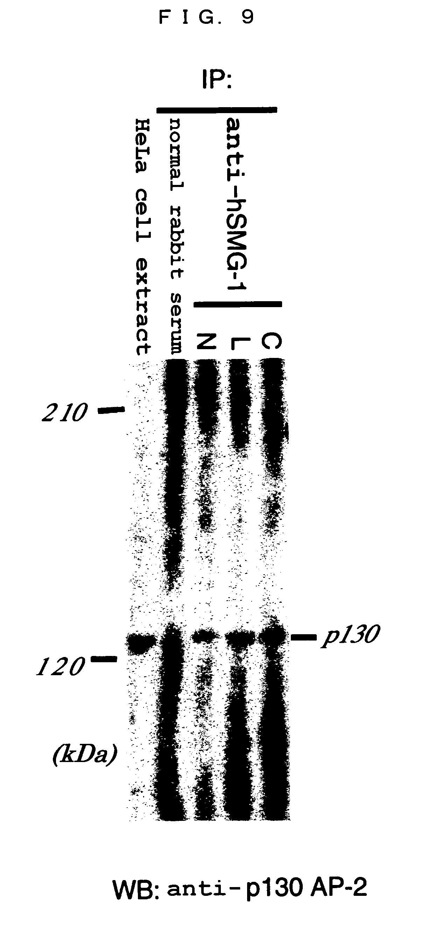

The results are shown in Figure 9. In Figure 9, the symbol "IP" means immunoprecipitation. The symbols "N", "L", and "C" indicate the results obtained by using antiserum N, antiserum L, and antiserum C, respectively. The symbol "WB" means Western blotting.

First, it was found that the antibodies recognized the translated product of AK026858.1, because the protein having a molecular weight of 130 kDa was specifically detected in the cell extract. Further, the same protein was detected in any case of immunoprecipitation using antiserum N, antiserum L, or antiserum C, but such a protein was not detected in the case of immunoprecipitation using the normal rabbit serum. From the results, it was found that the translated product of AK026858.1 was a binding protein specific to hSMG-1. From the molecular weight thereof, it was strongly suggested that the binding protein was the 130 kDa protein which may be phosphorylated in the hSMG-1 immunoprecipitate.

### INDUSTRIAL APPLICABILITY

According to the polypeptide of the present invention, a convenient system for screening an agent for treating and/or preventing a disease caused by one or more PTCs generated by a nonsense mutation can be provided. Further, the polynucleotide, the expression vector, the transformant, and the antibody according to the present invention are useful in producing the polypeptide of the present invention.

Although the present invention has been described with reference to specific embodiments, various changes and modifications obvious to those skilled in the art are possible without departing from the scope of the appended claims.

## Claims

(1) A polypeptide consisting of the amino acid sequence of SEQ ID NO: 2; (2) a polypeptide exhibiting an SMGBP1 activity and comprising the amino acid sequence of SEQ ID NO: 2; (3) a polypeptide exhibiting an SMGBP1 activity and comprising an amino acid sequence in which one or plural amino acids are deleted, substituted, and/or inserted at one or plural positions in the amino acid sequence of SEQ ID NO: 2; or (4) a polypeptide exhibiting an SMGBP1 activity and comprising an amino acid sequence having a 90% or more homology with that of SEQ ID NO: 2.

2. A polynucleotide encoding the polypeptide according to claim 1.

3. An expression vector comprising the polynucleotide according to claim 2.

4. A transformant comprising the polynucleotide according to claim 2.

5. An antibody or a fragment thereof, which binds to the polypeptide according to claim 1.

6. A knockout non-human animal or a cell thereof, wherein an expression of a gene encoding the polypeptide according to claim 1 is partially or completely suppressed.

7. A method for screening a substance which modifies an SMGBP1 activity of the polypeptide according to claim 1, comprising the steps of:
(1) bringing the polypeptide and SMG-1 into contact with a substance to be tested, under conditions such that the polypeptide exhibits the SMGBP1 activity in the absence of the substance to be tested; and
(2) analyzing whether or not the polypeptide exhibits the SMGBP1 activity.

8. A method for screening a substance which modifies an SMGBP1 activity of the polypeptide according to claim 1, comprising the steps of:
(1) bringing a cell capable of producing the polypeptide and SMG-1 into contact with a substance to be tested, under conditions such that the cell produces the polypeptide in the absence of the substance to be tested; and
(2) analyzing whether or not the polypeptide exhibits the SMGBP1 activity.

9. An agent for suppressing nonsense-mediated mRNA decay, comprising, as an active ingredient, a substance which modifies an SMGBP1 activity of the polypeptide according to claim 1.

10. An agent for treating and/or preventing a disease caused by a premature translation termination codon generated by a nonsense mutation, comprising, as an active ingredient, a substance which modifies an SMGBP1 activity of the polypeptide according to claim 1.

11. An agent for promoting nonsense-mediated mRNA decay, comprising as an active ingredient, a substance which modifies an SMGBP1 activity of the polypeptide according to claim 1.

12. A method for suppressing nonsense-mediated mRNA decay, comprising administering to a subject in need thereof a substance which modifies an SMGBP1 activity of the polypeptide according to claim 1 in an amount effective therefor.

13. A method for treating and/or preventing a disease caused by a premature translation termination codon generated by a nonsense mutation, comprising administering to a subject in need thereof a substance which modifies an SMGBP1 activity of the polypeptide according to claim 1 in an amount effective therefor.

14. A method for promoting nonsense-mediated mRNA decay, comprising administering to a subject in need thereof a substance which modifies an SMGBP1 activity of the polypeptide according to claim 1 in an amount effective therefor.

15. Use of a substance which modifies an SMGBP1 activity of the polypeptide according to claim 1, in the manufacture of an agent for suppressing nonsense-mediated mRNA decay.

16. Use of a substance which modifies an SMGBP1 activity of the polypeptide according to claim 1, in the manufacture of an agent for treating and/or preventing a disease caused by a premature translation termination codon generated by a nonsense mutation.

17. Use of a substance which modifies an SMGBP1 activity of the polypeptide according to claim 1, in the manufacture of an agent for promoting nonsense-mediated mRNA decay.
